(19) Europäisches Patentamt
European Patent Office
Office européen des brevets

(11) Veröffentlichungsnummer: **0 424 751 B1**

(12) **EUROPÄISCHE PATENTSCHRIFT**

(45) Veröffentlichungstag der Patentschrift: **21.06.95**

(51) Int. Cl.[6]: **C08F 246/00**, C08F 220/36, C08F 220/60, C09D 157/04, A61K 7/00, D06M 15/263, D06M 15/285, D06P 1/52

(21) Anmeldenummer: **90119571.9**

(22) Anmeldetag: **12.10.90**

# (54) Urethangruppen enthaltende Copolymerisate auf der Basis von ethylenisch ungesättigten Monomeren, Verfahren zu ihrer Herstellung und ihre Verwendung.

(30) Priorität: **21.10.89 DE 3935137**

(43) Veröffentlichungstag der Anmeldung:
**02.05.91 Patentblatt 91/18**

(45) Bekanntmachung des Hinweises auf die Patenterteilung:
**21.06.95 Patentblatt 95/25**

(84) Benannte Vertragsstaaten:
**BE DE ES FR GB IT**

(56) Entgegenhaltungen:
**EP-A- 0 011 806**
**EP-A- 0 084 227**
**EP-A- 0 121 230**
**EP-A- 0 252 526**

(73) Patentinhaber: **HOECHST AKTIENGESELLSCHAFT**

**D-65926 Frankfurt (DE)**

(72) Erfinder: **Huth, Hans-Ullrich, Dr.**
**Finkenweg 6**
**W-6073 Egelsbach (DE)**
Erfinder: **Zimmerschied, Klaus, Dr.**
**Fasanenweg 19**
**W-6200 Wiesbaden (DE)**

**EP 0 424 751 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services
(3.10/3.09/3.3.3)

## Beschreibung

Die Erfindung betrifft Copolymerisate auf der Basis von ethylenisch ungesättigten Monomeren, die Monomereinheiten aus grenzflächenaktiven Urethanderivaten mit ethylenisch ungesättigten Carboxy- oder Carbamidresten, ethylenisch ungesättigten Monomeren mit zur Salzbildung befähigten anionischen oder kationischen Resten, ethylenisch ungesättigten hydrophoben Monomeren sowie gegebenenfalls weiteren von den bereits genannten Monomeren verschiedenen ethylenisch ungesättigten Monomeren enthalten, Verfahren zu ihrer Herstellung, insbesondere durch radikalisch initiierte Emulsions-, Suspensions-, Perl- oder Lösungs-Copolymerisation und ihre Verwendung als sog. Verdickerkomponenten mit vorteilhafter rheologiemodifizierender Wirkung in wäßrigen Systemen, vorzugsweise in wäßrigen Kunststoffdispersionen, Anstrichfarben, Putzmörteln sowie Füllstoffe und/oder Pigmente enthaltenden wäßrigen Bindemittelsystemen.

Aus der EP-OS 197 635 ist die Herstellung von ethylenisch ungesättigten oberflächenaktiven und Urethangruppen enthaltenden Monomeren und deren Verwendung als sogenannte Makromere zur Copolymerisation und Herstellung von alkalilöslichen sogenannten Verdickerdispersionscopolymerisaten bereits bekannt. Die Herstellung dieser Urethangruppen enthaltenden monomeren Makromeren erfolgt durch Umsetzung von Aryl- oder Alkyl-polyalkylenglykoläthern an deren endständiger OH-Gruppe mit ethylenisch ungesättigten Isocyanaten, wie z.B. Isocyanatoethyl(meth-)acrylat. In den daraus hergestellten Copolymerisaten ist der Aryl- oder Alkyl-polyalkylenglykolätherrest als Seitenkette der Makromereinheit über eine Urethanbindung an die Hauptkette des Copolymerisatmoleküls gebunden. Die beschriebenen Verdickerprodukte konnten bisher jedoch keine praktische Bedeutung erlangen, da insbesondere ihre rheologiemodifizierenden Eigenschaften sich als unbefriedigend erwiesen haben.

Aus der EP-PS 84227 sind Acryloyl- und Alkylacryloylpolyalkoxycarbamate enthaltende flüssige Gemische und deren Verwendung für durch Strahlung härtbare Beschichtungen bekannt. Über eine Verwendbarkeit zur Herstellung von Copolymerisaten oder von Verdickercopolymerisaten enthält die Druckschrift keinerlei Angaben oder Hinweise.

Aus der EP-PS 121 230 sind Copolymerisate bekannt, die Monomereinheiten aus grenzflächenaktiven Crotonsäureestern sowie aus ungesättigten Carbonsäuren enthalten und als rheologiewirksame Verdicker in wäßrigen Systemen verwendbar sind. Die Copolymerisate können im einzelnen z.B. 1 bis 45 Gew.-% (Meth-)Acrylsäure, 30 bis 85 Gew.-% (Meth-)Acrylsäureester von ($C_1$-$C_{18}$)-Alkoholen und 0,5 bis 30 Gew.-% grenzflächenaktive Crotonsäureester von polyethoxylierten, langkettigen Alkoholen oder Alkylphenolen enthalten. Die Produkte sind im alkalischen pH-Bereich wasserlöslich und zeigen eine hohe Verdickerwirkung. Sie können z.B. in wäßrigen Dispersions-Glanzfarben zur Rheologiemodifizierung verwendet werden. Das Eigenschaftsbild bei Anstrichen mit solchermaßen verdickten Glanzfarben, wie z.B. Verlauf und Streichwiderstand des Anstrichmittels, Wirksamkeit bei niedriger Bindemittel-Konzentration bzw. Wirksamkeit in Mischung mit Pigmenten und/oder Füllstoffen sowie Wasserfestigkeit und Glanz des getrockneten Anstrichs kann jedoch die Anforderungen in der Praxis nur unzureichend erfüllen. So besitzen die in der EP-PS 121 230 beschriebenen und vorstehend genannten Verdicker zwar eine hohe Verdickerkapazität und können die Viskosität eines wäßrigen Dispersionslackes auf einem ausreichend hohen Wert bei hohem Schergefälle entsprechend dem in der Praxis erwünschten Streichwiderstand bei Applikation des Lackes mit dem Pinsel stabil halten. Die hierbei zutage tretenden rheologiemodifizierenden Wirkungen sind jedoch unzureichend bei Applikation des Dispersionsanstrichs mit einer Rolle, insbesondere an senkrechten Wänden und an Decken. Die genannten und makromere grenzflächenaktive Crotonestermonomereinheiten enthaltenden Verdicker besitzen nämlich im niederen Scherbereich (low-shear-viscosity) eine zu niedrige Viskosität, so daß Anstrichmittel, die diese Produkte enthalten, zum Spritzen neigen. Demgegenüber lassen vergleichbare Verdicker, die keine makromeren grenzflächenaktiven Crotonestermonomereinheiten in den Carboxylgruppen enthaltenden Copolymerisaten besitzen, den Streichwiderstand vermissen, d.h., ihre Viskosität im hohen Scherbereich (high-shear-viscosity) ist zu niedrig.

Der Erfindung lag somit die Aufgabe zugrunde, ein die Rheologie wäßriger Systeme beeinflussendes und in wäßrigen Zubereitungen verdickend wirkendes Copolymerisat verfügbar zu machen, welches in möglichst niedriger Konzentration bereits eine hohe Verdickerkapazität besitzt und in wäßrigen Systemen, wie z.B. in gel- oder pastenförmigen technischen Produkten, Dispersionsanstrichfarben, Kunststoffputzen, Dispersionsfliesenklebern, sowohl im niedrigen als auch im hohen Scherbereich ausreichende, vorzugsweise möglichst hohe und über größere Konzentrationsbereiche stabile Viskositäten erzeugt bzw. vermittelt und insbesondere als effektiver Verdicker zur Herstellung von nichtspritzenden Anstrichfarben verwendet werden kann.

Die vorstehend formulierte Aufgabe konnte nun überraschenderweise durch die Verwendung spezieller Copolymerisate auf der Basis von ethylenisch ungesättigten Monomeren gelöst werden, die makromere

Monomereinheiten aus grenzflächenaktiven Urethanderivaten mit ethylenisch ungesättigten Carboxy- oder Carbamidresten sowie Einheiten aus ethylenisch ungesättigten, salzbildungsfähigen Monomeren enthalten und durch radikalisch initiierte Copolymerisation nach üblichen Methoden hergestellt wurden. Eine besonders bevorzugte Copolymerisationsmethode ist hierbei die Emulsionscopolymerisation.

Die meisten der als Comonomere erfindungsgemäß eingesetzten grenzflächenaktiven makromeren Urethanderivate mit ethylenisch ungesättigten Carboxy- oder Carbamidresten, wie sie nachstehend durch die Formel I definiert sind, sind Gegenstand der am gleichen Tag eingereichten Patentanmeldung EP-A 0 424 750, auf die hiermit Bezug genommen wird.

Die seitenkettenständigen Urethanbindungen in den erfindungsgemäßen Copolymerisaten befinden sich aufgrund der Struktur der erfindungsgemäß eingesetzten comonomeren ethylenisch ungesättigten, grenzflächenaktiven makromeren Urethanderivate der Formel I nicht am hauptkettenständigen Anfang der Seitenketten, sondern im wesentlichen am Ende der Seitenketten, was zu einem überraschend vorteilhaften Eigenschaftsspektrum bei den Copolymerisaten, insbesondere hinsichtlich ihrer Verdickerwirkung, führt.

Erfindungsgemäß werden zur Copolymerisation besonders bevorzugt diejenigen erfindungsgemäßen monomeren ethylenisch ungesättigten, grenzflächenaktiven makromeren Urethanderivate der Formel I verwendet, die in ihrer monomeren Form bei 20 °C fest, wachsartig oder pastös sind.

Gegenstand der Erfindung sind daher Copolymerisate auf der Basis von ethylenisch ungesättigten Monomeren, die Monomereinheiten aus grenzflächenaktiven Urethanderivaten mit ethylenisch ungesättigten Carboxy- oder Carbamidresten sowie Einheiten aus ethylenisch ungesättigten, salzbildungsfähigen Monomeren enthalten und durch radikalisch initiierte Lösungs-, Emulsions-, Suspensions- oder Perl-Copolymerisation hergestellt worden sind oder deren Lösungen oder wäßrigen Dispersionen oder deren Salze oder Lösungen bzw. Dispersionen der Salze, dadurch gekennzeichnet, daß die Copolymerisatpartikel, bezogen in Gew.-% auf die Gesamtmenge an Monomereinheiten in dem Copolymerisat, aufgebaut sind aus

a) 25 bis 85 Gew-% ethylenisch ungesättigten hydrophoben Monomeren aus der Gruppe Vinylester von ($C_1$-$C_{18}$)-Monocarbonsäuren, vorzugsweise Vinylacetat, Vinylpropionat, Vinylversatat, Vinyllaurat, Vinylstearat, (Meth-)Acrylester von ($C_1$-$C_{22}$)-Alkoholen, vorzugsweise Methylmethacrylat, Butylmethacrylat, Oktylmethacrylat, Ethylacrylat, Isobutylacrylat, 2-Ethylhexylacrylat, Vinylaromaten mit bis zu 18 C-Atomen, vorzugsweise Styrol und Vinyltoluol, Vinylchlorid, Ethylen, (Meth-)Acrylnitril, Diester von Maleinsäure und/oder Fumarsäure mit ($C_1$-$C_{22}$)-Alkoholen, Vinylpyrrolidon,

und

b) 1 bis SO Gew.-% ethylenisch ungesättigten, salzbildungsfähigen Monomeren mit funktionellen anionischen Resten aus der Gruppe -COOH, Sulfonsäuren bzw. Sulfonsäurederivate oder Phosphonsäuren bzw. Phosphonsäurederivate, vorzugsweise Carbonsäurereste, insbesondere Monomere aus der Gruppe ethylenisch ungesättigter ($C_3$-$C_5$)-Mono- oder Dicarbonsäuren, wie Acrylsäure, Methacrylsäure, Crotonsäure, Itakonsäure, Maleinsäure, Fumarsäure sowie der Halbester der zweibasigen Carbonsäuren mit geradkettigen oder verzweigten ($C_1$-$C_8$)-Alkoholen, ferner Monomere aus der Gruppe Vinylsulfonsäure, (3-Sulfopropyl)-methacrylsäureester, Acrylamidomethylpropansulfonsäure, Vinylphosphonsäure, Acrylamidomethylpropanphosphonsäure bzw. deren Salze, vorzugsweise Alkali- oder Ammoniumsalze,

oder

anstelle von anionischen Monomeren, ethylenisch ungesättigten, salzbildungsfähigen Monomeren mit funktionellen kationischen Resten aus der Gruppe $-NR^5R^6$, wobei $R^5$ und $R^6$ gleich oder verschieden sein können und H oder ($C_1$-$C_{18}$)-Alkyl bedeuten, oder $R^5$ und $R^6$ gemeinsam mit N gegebenenfalls einen fünf- bis siebengliedrigen heterocyclischen Ring bilden, vorzugsweise Dimethylaminoneopentyl-(meth-)acrylat, Dimethylaminopropyl(meth-)acrylamid, Dimethylaminoethyl(meth-)acrylat, 2-N-Morpholinoethyl(meth-)acrylat, tert.-Butylaminoethyl(meth-)acrylat, ethylenisch ungesättigten ($C_3$-$C_{18}$)-aliphatischen primären Aminen oder sekundären Aminen mit einem ($C_1$-$C_{18}$)-Alkylrest oder tertiären Aminen mit zwei ($C_1$-$C_{18}$)-Alkylresten,

und

c) 0,1 bis 30 Gew.-% ethylenisch ungesättigten grenzflächenaktiven Urethanderivaten der Formel I,

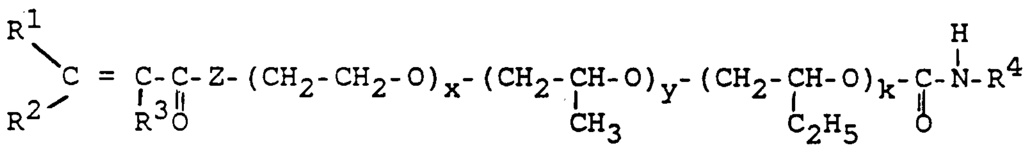

(I)

worin die Reste $R^1$ bis $R^4$ und Z sowie die Zahlenindices x, y und k folgendes bedeuten:

$R^1$,$R^2$,$R^3$, die gleich oder verschieden sein können,
= H, $-CH_3$, -COOH, $-CH_2$-COOH, vorzugsweise H, $-CH_3$,

Z = Sauerstoff oder NH, vorzugsweise Sauerstoff,

x,y,k, die gleich oder verschieden sein können,
= 0 bis 100 mit der Maßgabe, daß $x+y+k \geqq 2$, vorzugsweise $x+y = 2$ bis 30 bei $k = 0$,

$R^4$ = gegebenenfalls substituiertes ($C_1$-$C_{30}$)-Alkyl, vorzugsweise ($C_1$-$C_{18}$)-Alkyl, gegebenenfalls substituiertes ($C_6$-$C_{10}$)-Aryl, gegebenenfalls substituiertes ($C_7$-$C_{30}$)-Aralkyl, gegebenenfalls substituiertes ($C_5$-$C_8$)-Cycloalkyl, einen gegebenenfalls substituierten 5- bis 7-gliedrigen Heterocyclus,

und wobei besonders bevorzugte Urethanderivate solche sind, bei denen in Formel I $R^1$, $R^2$ = H, $R^3$ = $-CH_3$ oder $R^1$, $R^3$ = H, $R^2$ = $-CH_3$, Z = Sauerstoff und $x+y = 2$ bis 30 bei $k = 0$ bedeutet,
und
d) 0 bis 10 Gew.-% weiteren, von a) bis c) verschiedenen ethylenisch ungesättigten Monomeren mit funktionellen Resten aus der Gruppe -OH,

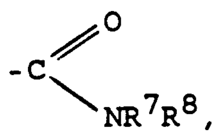

wobei $R^7$ und $R^8$ gleich oder verschieden sein können und für H, ($C_1$-$C_6$)-Alkyl, ($C_2$-$C_8$)-Alkoxyalkyl, ($C_5$-$C_7$)-Cycloalkyl oder ($C_6$-$C_{18}$)-Aralkyl stehen, oder $R^7$ und $R^8$ gemeinsam mit N gegebenenfalls einen fünf- bis siebengliedrigen heterocyclischen Ring bilden, vorzugsweise ethylenisch ungesättigten Hydroxyalkylestern der (Meth-)Acrylsäure, insbesondere Hydroxyethyl(meth-)acrylat, Hydroxypropyl(meth-)acrylat, Polyalkylenoxidestern der (Meth-)Acrylsäure, insbesondere mit 2 bis 50 Ethylenoxideinheiten und/oder 2 bis 50 Propylenoxideinheiten, wobei die endständigen OH-Gruppen der Ester- bzw. der Polyalkylenglykolätherreste auch veräthert oder verestert sein können, ethylenisch ungesättigten Amiden, insbesondere (Meth-)Acrylamid, N-Methylacrylamid, N,N-Dimethyl(meth-)acrylamid, N-Butylmethacrylamid, N-Cyclohexylmethacrylamid, N-Benzylmethacrylamid, N-Methylol(meth-)acrylamid, N-Butoxymethyl(meth-)acrylamid,
und
e) 0 bis 5 Gew.-% ethylenisch ungesättigten Carbonylverbindungen, vorzugsweise aus der Gruppe Vinylmethylketon, Acrolein, Crotonaldehyd, Allylacetoacetat, Acetoacetoxyethyl(meth-)acrylat,
und
f) 0 bis 5 Gew.-% mehrfach ethylenisch ungesättigten bzw. mehrfachfunktionellen und zur Vernetzung befähigten Monomeren, vorzugsweise aus der Gruppe Divinylbenzol, Diallylphthalat, Butandioldiacrylat, Triethylenglykoldimethacrylat, Allylmethacrylat, Bisphenol-A-diethylenglykoldimethacrylat, Triallylcyanurat, Methylen-bis-methacrylamid,
und
g) 0 bis 5 Gew.-% Molekulargewichtsreglern aus der Gruppe Dodecylmerkaptan, Tetrachlorkohlenstoff, $\alpha$-Methylstyrol, Toluol, Bromtrichlormethan, Tetrakismerkaptoacetylpentaerythrit, Thioglykolsäure,
und bei Vorliegen wäßriger Dispersionen bzw. der daraus isolierten Copolymerisate diese außerdem
h) 0,1 bis 10 Gew.-%, bezogen auf die Gesamtmenge aller Monomereinheiten in dem Copolymerisat, Emulgatoren und/oder gegebenenfalls Schutzkolloide, vorzugsweise aus der Gruppe der anionischen oder kationischen oder zwitterionischen und/oder insbesondere der nichtionogenen Tenside und/oder Schutzkolloide, enthalten.

Die Herstellung der erfindungsgemäß als Comonomere zur Copolymerisation eingesetzten und vorstehend unter der Comonomereinheitengruppe c) beschriebenen ethylenisch ungesättigten, grenzflächenaktiven Urethanderivate der Formel I,

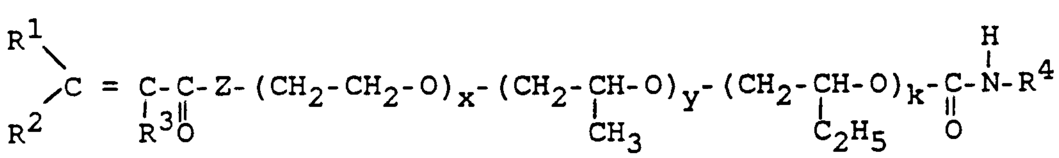
(I)

worin $R^1$ bis $R^4$, Z, x, y und k die vorstehend genannten Bedeutungen haben, erfolgt durch Umsetzung von Isocyanaten mit Hydroxypolyoxyalkylenoxycarbonylalkenen oder mit N-(Hydroxypolyoxyalkylen)-alkencarboxamiden, indem man Isocyanate der Formel II,

$R^4$—NCO (II),

worin $R^4$ die Bedeutung wie in Formel I hat, mit äquimolaren Mengen von Hydroxyverbindungen der Formel III,

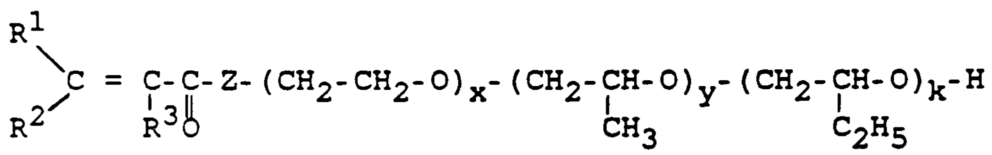

(III)

worin $R^1$, $R^2$, $R^3$, Z, x, y und k die Bedeutung wie in Formel I haben, umsetzt.

Bevorzugte Verbindungen der Formel III sind Polyalkylenglykolmonoester von ethylenisch ungesättigten Carbonsäuren, vorzugsweise von Acrylsäure, Methacrylsäure oder Crotonsäure.

Die Umsetzung der Komponenten der Formeln II und III erfolgt bevorzugt in Substanz oder in inerten organischen Lösungsmitteln oder gegebenenfalls in unter den Umsetzungsbedingungen sich inert verhaltenden anderen zur erfindungsgemäßen Copolymerisation verwendeten copolymerisationsfähigen, ethylenisch ungesättigten Monomeren (sogenannte Reaktivverdünner) in Abwesenheit von Wasser, vorzugsweise bei Temperaturen zwischen 0 °C und Raumtemperatur, oder bei einer höheren Temperatur, vorzugsweise bis zu 60 °C.

Als inerte organische Lösungsmittel kommen die bei organischen Synthesen mit Isocyanaten in wasserfreiem Medium üblicherweise verwendeten inerten Lösungsmittel zur Anwendung, sofern sie u.a. die Erfordernisse hinsichtlich Lösewirkung und Siedebereich erfüllen können.

Bevorzugte inerte Lösungsmittel sind z.B. Toluol, Tetrahydrofuran (THF), Ethylacetat und Hexan.

In manchen Fällen können Reaktivverdünner als Lösungsmittel vorteilhaft sein. Reaktivverdünner sind copolymerisationsfähige Monomere, die sich unter den Synthesebedingungen der Urethangruppenbildung inert verhalten, sich aber später unter geeigneten Polymerisationsbedingungen mit den ungesättigten Urethanderivaten der Formel I copolymerisieren lassen. Bevorzugte inerte Reaktivverdünner sind demzufolge solche, die zur erfindungsgemäßen Copolymerisation als Comonomere mitverwendet werden können, wie z.B. (Meth-)Acrylsäureester, Styrol und Vinylester, wobei man bei deren Verwendung vorzugsweise unterhalb der Sättigungskonzentration der Reaktanten arbeitet.

Gegebenenfalls wird bei der Isocyanataddition ein Katalysator mitverwendet, u.a. um die Reaktionstemperatur möglichst niedrig halten zu können, was insbesondere bei der Verwendung von Reaktivverdünnern vorteilhaft sein kann. Bevorzugte Katalysatoren sind organische Zinnverbindungen, die vorzugsweise in einem inerten organischen Lösungsmittel gelöst eingesetzt werden.

Besonders bevorzugt ist die Verwendung von Dibutylzinndilaurat, gegebenenfalls in Kombination mit tert.-Butylbrenzkatechin.

Die erfindungsgemäßen Copolymerisate werden bei teilweiser oder vollständiger Neutralisation bzw. teilweiser oder vollständiger Überführung ihrer salzbildungsfähigen Reste in die Salzform zunehmend bis vollständig wasserlöslich bzw. kolloidal wasserlöslich oder in Wasser kolloidal dispergierbar. In solchermaßen teilweise oder vollständig neutralisierter Form können sie als Verdicker wäßriger Systeme mit überraschend vorteilhaften rheologiemodifizierenden Eigenschaften in dem vorstehend beschriebenen Sinne wirken bzw. verwendet werden.

Weiterer Gegenstand der Erfindung sind daher die vorstehend beschriebenen erfindungsgemäßen Copolymeriste in ihrer teilweise oder vollständig neutralisierten wasserlöslichen oder in Wasser kolloidal dispergierbaren Form, wobei die Neutralisation der anionischen Copolymerisate mit Basen und die der kationischen Copolymerisate mit Säuren erfolgte. Bevorzugt erfolgt die partielle oder vollständige Neutralisation erfindungsgemäßer Copolymerisate in deren wäßriger Dispersionsform. In manchen Fällen kann die Neutralisationsreaktion auch vorteilhaft in Lösungen der Copolymerisate in organischen Lösungsmitteln durchgeführt werden.

Eine bevorzugte Neutralisationsmethode besteht ferner u.a. darin, daß man z.B. die anionischen, nicht neutralisierten Copolymerisate in ihrer wasserunlöslichen Säureform als Lösungen in einem geeigneten organischen Lösungsmittel oder insbesondere als niedrigviskose wäßrige Dispersion dem zu verdickenden

wäßrigen bzw. wasserhaltigen System zumischt und das resultierende Gemisch anschließend durch Basenzusatz teilweise oder vollständig neutralisiert, wodurch die Copolymerisate unter Entfaltung ihrer Verdickungswirkung und rheologiemodifizierenden Wirkung in eine wasserlösliche Salzform umgewandelt werden. In analoger Weise können die kationischen, nicht neutralisierten Copolymerisate in ihrer wasserunlöslichen basischen Form bei ihrer Anwendung durch Umsetzung mit Säuren in wasserlösliche Salzformen umgewandelt werden, in denen sie die erfindungsgemäße Verdickungswirkung sowie die rheologiemodifizierende Wirkung entfalten können.

Wie bereits vorstehend ausgeführt, können die erfindungsgemäßen Copolymerisate z.B. durch übliche, radikalisch initiierte Lösungscopolymerisation in üblichen organischen Lösungsmitteln, oder vorzugsweise in Form einer wäßrigen Copolymerisatdispersion durch übliche radikalisch initiierte Emulsionscopolymerisation hergestellt werden. Dabei liegt der Feststoffgehalt von solchermaßen hergestellten wäßrigen Copolymerisatdispersionen vorzugsweise im Bereich von 10 bis 55 Gew.-% Feststoff, bezogen auf die Dispersion.

Bei der bevorzugten Emulsionscopolymerisation werden ferner die vorstehend als comonomere Komponenten c) genannten monomeren, grenzflächenaktiven Urethanderivate der Formel I vorzugsweise in der Wasserphase vorgelegt.

Bei der Herstellung erfindungsgemäßer Copolymerisate durch radikalisch initiierte Lösungscopolymerisation, vorzugsweise in üblichen organischen Lösungsmitteln und nach üblichen Methoden, werden die resultierenden Copolymerisate vorzugsweise in feindisperser Pulver- oder Granulatform isoliert bzw. erhalten und gegebenenfalls durch nachfolgende Trocknung von flüchtigen Bestandteilen befreit.

Bei der Herstellung erfindungsgemäßer Copolymerisate durch Emulsionscopolymerisation kann ebenfalls nach üblichen Methoden verfahren werden. Dabei können übliche ionogene und/oder nichtionogene Emulgatoren zur Emulgierung der Monomeren und Stabilisierung der resultierenden Latices eingesetzt werden.

Bevorzugte anionische Emulgatoren sind bei der Herstellung anionischer Emulsionscopolymerisate z.B. grenzflächenaktive Alkylsulfate, Alkylsulfonate, Alkylarylsulfate, Alkylarylsulfonate, Alkali- und/oder Ammoniumsalze von Alkyl- bzw. Alkylarylglykolethersulfaten.

Bevorzugte nichtionogene Emulgatoren sind z.B. tensioaktive oxethylierte Fettalkohole oder oxethylierte Alkylphenole.

Bevorzugte Emulgatoren sind bei der Herstellung kationischer Emulsionscopolymerisate z.B. nichtionogene Emulgatoren und/oder kationische Tenside, vorzugsweise aus der Gruppe der Fettaminoxethylate, der quartären Alkylammoniumverbindungen und der Salze von Fettaminen.

Weitere Gegenstände der Erfindung sind daher ferner Verfahren zur Herstellung der vorstehend beschriebenen erfindungsgemäßen Copolymerisate durch radikalisch initiierte Emulsions- oder Lösungscopolymerisation der genannten ethylenisch ungesättigten copolymerisationsfähigen Monomeren in den erforderlichen Mischungsverhältnissen nach an sich bekannten Methoden, sowie gegebenenfalls nachfolgender teilweiser oder vollständiger Neutralisation der salzbildungsfähigen Monomereinheiten unter Bildung von in Wasser kolloidal dispergierbaren oder wasserlöslichen Copolymerisatsalzen, wobei die Neutralisation von anionischen Copolymerisaten durch Zusatz von Basen bzw. basischen Verbindungen und die Neutralisation von kationischen Copolymerisaten durch Zusatz von Säuren bzw. sauren Verbindungen erfolgt.

Eine besonders bevorzugte Verfahrensweise ist die Emulsionscopolymerisation, wobei wiederum vorzugsweise die vorstehend als comonomere Komponenten c) beschriebenen ethylenisch ungesättigten grenzflächenaktiven Urethanderivate der Formel I in der wäßrigen Phase vorgelegt werden. Der Copolymerisatanteil in den solchermaßen durch Emulsionscopolymerisation hergestellten wäßrigen erfindungsgemäßen Copolymerisatdispersionen beträgt vorzugsweise 10 bis 55 Gew.-%, insbesondere 25 bis 50 Gew.-%

Im Falle der Emulsionscopolymerisation kann es außerdem von erheblicher Bedeutung sein, ob die Monomeren als solche oder in wäßriger Emulsionsform der Copolymerisationsreaktion zugeführt bzw. zudosiert werden. Einen ähnlich bedeutsamen Einfluß hat ferner die Art der Emulgatorzugabe. So können in Abhängigkeit davon, ob der Emulgator in der wäßrigen Phase vorgelegt oder während der Copolymerisation zudosiert wird, große Unterschiede hinsichtlich der Teilchengröße der Copolymerisate, der Teilchengrößenverteilung sowie der Stabilität der Copolymerisatdispersionen auftreten.

Die Mengenanteile der vorstehend beschriebenen comonomeren Komponenten a) bis f) sowie der weiteren Komponenten g) und h) können bei allen erfindungsgemäßen Copolymerisationsverfahren innerhalb eines relativ weiten Rahmens variiert werden und betragen, bezogen in Gew.-% auf die Gesamtmenge aller Comonomeren a) bis f), jeweils vorzugsweise,

für a) 25 bis 85 Gew.-%, insbesondere 40 bis 80, besonders bevorzugt 50 bis 75 Gew.-%,
für b) 1 bis 50 Gew.-%, insbesondere 5 bis 45, besonders bevorzugt 8 bis 40 Gew.-%,
für c) 0,1 bis 30 Gew.-%, insbesondere 1 bis 25, besonders bevorzugt 3 bis 20 Gew.-%,
für d) 0 bis 10 Gew.-%, insbesondere 0,1 bis 8, besonders bevorzugt 1 bis 6 Gew.-%,

für e) 0 bis 5 Gew.-%, insbesondere 0,05 bis 3, besonders bevorzugt 0,1 bis 2 Gew.-%, und

für f) 0 bis 5 Gew.-%, insbesondere 0,01 bis 1, besonders bevorzugt 0,1 bis 0,5 Gew.-%.

Die Komponenten g) und h) werden, bezogen in Gew.-% auf die Gesamtmenge aller Comonomeren a) bis f), jeweils vorzugsweise eingesetzt in Mengen von

g) 0 bis 5 Gew.-%, insbesondere 0,05 bis 3, besonders bevorzugt 0,1 bis 2 Gew.-%, und,

bei der Emulsionscopolymerisation,

h) 0,1 bis 10 Gew.-%, insbesondere 0,1 bis 6, besonders bevorzugt 0,2 bis 3 Gew.-%.

Zur Initiierung der Emulsionscopolymerisation werden vorzugsweise übliche, Radikalketten startende, wasserlösliche Initiatoren in Mengen von 0,01 bis 2 Gew.-%, bezogen auf die Gesamtmenge aller Comonomeren, eingesetzt.

Besonders geeignet hierfür sind z.B. Alkali- oder Ammoniumpersulfat, $H_2O_2$, tert.-Butylhydroperoxid, übliche Redoxkatalysatoren, 4,4'-Azobis-(4-cyanovaleriansäure), 2,2'-Azobis-(N,N'-dimethylenisobutyramidin)-dihydrochlorid, 2,2'-Azobis-(2-amidinopropan)-dihydrochlorid, wobei die beiden letzteren Initiatoren insbesondere zur Herstellung von kationischen Copolymerisaten geeignet sind, ferner energiereiche Strahlen sowie übliche Photoinitiatoren.

Zur Steigerung der Verdickerkapazität bzw. zur weiteren Beeinflussung der rheologischen Eigenschaften und Wirksamkeit von gelösten erfindungsgemäßen Copolymerisaten in wäßrigen Systemen kann es in manchen Fällen vorteilhaft sein, wenn bei der Copolymerisation mehrfach ethylenisch ungesättigte Monomere, vorzugsweise aus der vorstehend beschriebenen monomeren Komponentengruppe f), als Comonomere mitverwendet werden, um höhere Molekulargewichte bei den Copolymerisaten zu erzielen. Als solche vernetzend wirkenden und zu Molekulargewichtserhöhungen führenden Comonomeren werden vorzugsweise z.B. Diallylphthalat, Divinylbenzol, Allylmethacrylat oder Ethylenglykoldimethacrylat verwendet. Die eingesetzten Mengen können vorzugsweise in einem Bereich zwischen 0 und 5 Gew.-%, bezogen auf die gesamte Comonomerenmenge, liegen und können insbesondere 0,01 bis 1 Gew.-%, besonders bevorzugt 0,1 bis 0,5 Gew.-%, betragen. Die mehrfach ethylenisch ungesättigten Comonomeren können bei der Copolymerisation Molekülverzweigungen und Netzwerke bilden, die nach der partiellen oder vollständigen Neutralisation des Copolymerisats zur Bildung von Gelstrukturen und damit zur Ausbildung spezifischer und für manche Anwendungen vorteilhafter rheologischer Eigenschaftsprofile in wäßrigen Systemen führen können.

Durch die Mitverwendung von Molekulargewichtsreglern aus der vorstehend beschriebenen Komponentengruppe g) während der Copolymerisation können die Molekulargewichte der Copolymerisate erniedrigt werden. Mit sinkenden Molekulargewichten der Copolymerisate vermindert sich aber die Verdickungswirkung neutralisierter Copolymerisate in wäßrigen Systemen und die Viskositäten vergleichbarer wäßriger Lösungen von teilweise oder vollständig neutralisierten Copolymerisaten werden mit fallenden Molekulargewichten niedriger, vergleichsweise zu Copolymerisaten, die ohne Molekulargewichtsregler hergestellt wurden. Man kann jedoch mit Hilfe der Molekulargewichtsregler die Möglichkeiten verbessern, das Gleichgewicht zwischen der Viskosität der neutralisierten Copolymerisate in wäßrigen Systemen bei hoher und bei niedriger Scherbeanspruchung zu harmonisieren und für spezielle Anwendungen gezielt einzustellen. Obgleich die Molekulargewichte erfindungsgemäßer Copolymerisate, insbesondere nach oben hin, keiner besonderen Begrenzung unterliegen, so liegen sie im unteren Bereich jedoch vorzugsweise oberhalb von 10000 g/mol, insbesondere oberhalb von 30000 g/mol.

Als Molekulargewichtsregler bei der Copolymerisation kommen prinzipiell alle Verbindungen infrage, welche Radikale übertragende Eigenschaften besitzen. Vorzugsweise werden dafür die vorstehend unter der Komponentengruppe g) beschriebenen Verbindungen verwendet. Besonders bevorzugt werden monofunktionelle oder mehrfachfunktionelle Merkaptane verwendet, wie z.B. Dodecylmerkaptan, Tetrakismerkaptoacetylpentaerythrit, Thioglykolsäure.

Bevorzugt sind ferner z.B. auch $\alpha$-Methylstyrol, Toluol, Bromtrichlormethan, Tetrachlorkohlenstoff. Die eingesetzte Menge an Regler kann vorzugsweise im Bereich zwischen 0 und 5 Gew.-%, bezogen auf die gesamte Comonomerenmenge, liegen und kann insbesondere 0,05 bis 3 Gew.-%, besonders bevorzugt 0,1 bis 2 Gew.-%, betragen.

Die erfindungsgemäßen Copolymerisate besitzen in ihrer teilweise und/oder vollständig neutralisierten Form in wäßrigen Systemen überraschend hohe Verdickerkapazitäten. Besonders vorteilhaft und überraschend ist ihre Eigenschaft, die hohen Viskositäten in wäßrigem Milieu sowohl im niedrigen als auch im hohen Scherbereich zu entfalten, was sie u.a. insbesondere für die Herstellung von nichtspritzenden Anstrichfarben geeignet macht. Ein weiterer wichtiger Vorteil besteht darin, daß die erfindungsgemäßen Copolymerisate sowohl im sauren als auch im alkalischen pH-Wert-Bereich hydrolysebeständig sind. Das vorteilhafte Eigenschaftsspektrum der erfindungsgemäßen Copolymerisate ermöglicht deren Einsatz als Verdicker in geringeren Aufwandmengen als beim vergleichbaren Einsatz von vergleichbaren Copolymerisaten, die zwar ebenfalls Methacrylsäure- und Acrylester-einheiten aber keine Monomereinheiten aus grenzflächenaktiven Urethanderivaten der Formel I, wie vorstehend unter der Komponentengruppe c) beschrieben, enthalten, um wäßrigen Systemen bestimmte rheologische Eigenschaften zu vermitteln. Durch die hohe spezifische und über einen breiten Anwendungsbereich effektive Verdickerwirkung der erfindungsgemäßen Produkte können die erforderlichen Wirkungen bereits mit relativ geringen Einsatzmengen in den zu verdickenden Formulierungen erzielt werden, so daß nachteilige Auswirkungen auf andere Eigenschaften bei den fertigen Formulierungen, wie z.B. die Wasserempfindlichkeit von daraus hergestellten und getrockneten Farbanstrichen, praktisch nicht auftreten können, im Gegensatz zu üblichen Verdickern, die für vergleichbare Rheologieeinstellungen in deutlich höheren Aufwandmengen eingesetzt werden müssen, was im allgemeinen häufig zu den bekanntermaßen störenden und schädlichen Wasserempfindlichkeiten bei den Endprodukten, z.B. bei getrockneten Farbanstrichen, führen kann.

Die Wirksamkeit erfindungsgemäßer Copolymerisate als Verdicker für wäßrige Systeme ergibt sich vorzugsweise in deren teilweise oder vollständig neutralisierter Form, wobei die Neutralisation bei anionischen Copolymerisaten durch Zusatz von anorganischen oder organischen Basen, und bei kationischen Copolymerisaten durch Zusatz von organischen oder anorganischen Säuren, jeweils nach Maßgabe der Stöchiometrie, erfolgt. Durch die Neutralisation werden die in ihrer nichtneutralisierten Form im allgemeinen wasserunlöslichen erfindungsgemäßen Copolymerisate in eine wasserlösliche oder eine kolloidal wasserlösliche bzw. wasserdispergierbare Form übergeführt, in der sie ihre Verdickerwirkung in wäßrigen Systemen entfalten und diesen Systemen spezielle und stabile rheologische Eigenschaften verleihen können. Besonders bevorzugt sind bei den anionischen Copolymerisaten die Alkali-, Ammonium- und Aminsalze bzw. bei teilweiser Neutralisation die entsprechenden Partialsalze.

Bei den kationischen Copolymerisaten sind besonders bevorzugt die mineralsauren sowie die mono- oder poly-carbonsauren Salze.

Besonders bevorzugt sind ferner die anionischen Copolymerisate bzw. deren teilneutralisierte und vollneutralisierte wasserlöslichen Salzformen, einschließlich deren Verwendung als Verdicker und rheologiemodifizierende Komponenten in wäßrigen Systemen.

Der Einsatz der erfindungsgemäßen Copolymerisate als Verdicker für wäßrige Systeme erfolgt bevorzugt und besonders vorteilhaft in der Weise, daß man sie in ihrer nicht neutralisierten Form als niedrigviskose Copolymersat-Lösung in einem geeigneten Lösungsmittel oder als wenig viskose wäßrige Copolymerisat-Dispersion dem zu verdickenden wäßrigen System beimischt und anschließend das resultierende Gemisch, einschließlich aller gegebenenfalls mitverwendeten Hilfsstoffe, Zusatzmittel, Pigmente, Lackbestandteile, etc., die bei den einzustellenden pH-Werten beständig sein müssen, bei anionischen Verdickern durch Basenzusatz und bei kationischen Verdickern durch Säurezusatz teilweise oder vollständig neutralisiert und gegebenenfalls leicht alkalisch bei den anionischen Verdickern bzw. leicht sauer bei den kationischen Verdickern einstellt. Der bevorzugte pH-Bereich bei der Neutralisation mit Basen liegt bei pH 5 bis 10,5, vorzugsweise 8 bis 9,5, und bei der Neutralisation mit Säuren bei 2 bis 6,5, vorzugsweise 3 bis 5,5.

Besonders bevorzugt werden die nicht neutralisierten Copolymerisate in Form von wäßrigen Copolymerisat-Dispersionen den zu verdickenden wäßrigen Systemen beigemischt.

Bevorzugtes Einsatzgebiet für die erfindungsgemäßen Copolymerisate ist ihre Verwendung als Verdikker zum Verdicken und zur Viskositätseinstellung von wäßrigen Lösungen und von dispersen wäßrigen Systemen, vorzugsweise z.B. von wäßrigen Dispersionsanstrichfarben, Dispersionsglanzlacken, Textildruckpasten, Papierdruckpasten, bioziden Wirkstofflösungen und Wirkstoffdispersionen, insbesondere für den Pflanzenschutz und für die Schädlingsbekämpfung, Flüssigdüngemitteln, Emulsionsreinigern, Abbeizpasten, Enteisungsmitteln und kosmetischen Zubereitungen.

Eine weitere interessante Verwendung der erfindungsgemäßen Copolymerisate ist deren Einsatz als durch alkalische oder saure Flotten leicht auswaschbare Schlichtemittel in der Textilindustrie bzw. als Schlichtemittelkomponenten.

In wäßrigen Dispersionsanstrichfarben und in wäßrigen Dispersionsglanzlacken führt der Einsatz erfindungsgemäßer Copolymerisate z.B. besonders vorteilhaft zur Ausbildung der vom Anwender gewünschten rheologischen Eigenschaften, wie hoher high-shear-viscosity und hoher low-shear-viscosity, wobei die letztere Eigenschaft für nichtspritzende Anstrichformulierungen von Bedeutung ist. Unter hoher high-shear-viscosity werden dabei Werte , ermittelt an z.B. 5 gew.-%igen wäßrigen Verdickerlösungen, zwischen 0,01 und 1 Pa•s bei einer Schergeschwindigkeit von $D = 10000 \cdot s^{-1}$, und unter hoher low-shear-viscosity Werte zwischen 0,1 und 100 Pa•s bei $D = 0,1 \cdot s^{-1}$ verstanden.

Die Einsatzmengen an den erfindungsgemäßen Copolymerisaten sind nicht kritisch. Sie liegen beim Einsatz als Verdicker jedoch vorzugsweise im Bereich von 0,01 bis 5 Gew.-% Copolymerisat, bezogen auf das zu verdickende wäßrige System.

Die Erfindung wird durch die nachfolgenden Beispiele näher erläutert.

**Beispiel 1**

Herstellung eines Emulsionscopolymerisats mit der comonomeren Formel I-Urethanverbindung

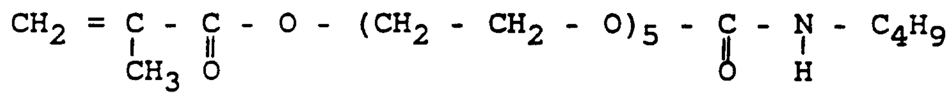

In einem (2 l)-Dreihalskolben mit Rührer, Rückflußkühler und Innenthermometer werden 28 g des Na-Salzes eines Alkylarylpolyglykolethersulfats (50 gew.-%ig) in 738 g entsalztem Wasser (= E-Wasser) gelöst und unter Rühren auf 80 °C erwärmt. Von einer Initiatorlösung, bestehend aus 0,25 g Ammoniumpersulfat in 50 g E-Wasser, werden 10 ml zugesetzt sowie 50 g einer Monomerenmischung, bestehend aus 252 g Ethylacrylat (= 72 Gew.%), 63 g Methacrylsäure (= 18 Gew.-%) und 35 g der oben angegebenen monomeren Formel I-Urethanverbindung (= 10 Gew.-%, jeweils bezogen auf die gesamte Monomerenmenge). Anschließend wird der Rest der Monomerenmischung, nach einer halben Stunde beginnend, während zwei Stunden zusammen mit dem Rest der Initiatorlösung zudosiert. Nach dem Ende der Zudosierung wird unter Fortsetzung des Rührens noch eine weitere Stunde nachgeheizt und der Ansatz anschließend auf Raumtemperatur gebracht. Der Feststoffgehalt der resultierenden Dispersion wird auf 30 Gew.-% eingestellt.

Durch teilweise oder vollständige Neutralisation der Dispersion mit wäßriger Natronlauge oder mit wäßrigem Ammoniak kann der Copolymerisatanteil in eine wasserlösliche Form mit den gewünschten, wäßrige Systeme verdickenden und rheologiemodifizierenden Eigenschaften übergeführt werden. Durch Wasserentzug nach üblichen Methoden, vorzugsweise z.B. durch Ausfällen und/oder Trocknung, können aus der Dispersion oder der Lösung die copolymeren Bestandteile in trockener Form, vorzugsweise pulverförmig oder granulatförmig, erhalten werden. Die Viskositätszahlen, gemessen im Epprecht-Rheomat an dem vollständig mit Natronlauge neutralisierten Copolymerisat in 5 gew.-%iger wäßriger Lösung bei 20 °C, betragen $10 \cdot 10^{-2}$ Pa·s bei D = $10000 \cdot s^{-1}$ und $12 \cdot 10^{-1}$ Pa·s bei D = $0,1 \cdot s^{-1}$.

**Beispiel 2**

Beispiel 1 wird wiederholt mit der Abänderung, daß das eingesetzte Monomerengemisch aus 50 Gew.-% Ethylacrylat, 40 Gew.-% Methacrylsäure, 10 Gew.-% der monomeren Formel I-Urethanverbindung

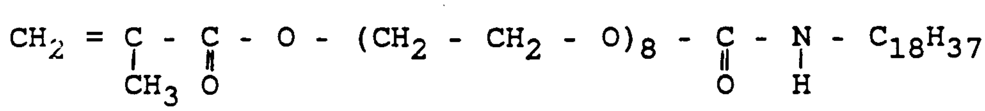

sowie zusätzlich 0,1 Gew.-%, bezogen auf die gesamte Monomerenmenge, des Molekulargewichtsreglers n-Dodecylmerkaptan besteht.

Der Feststoffgehalt der resultierenden Dispersion wird auf 30 Gew.-% eingestellt.

Die teilweise oder vollständige Neutralisation des Copolymerisatanteils sowie gegebenenfalls dessen Isolierung in trockener Form kann analog Beispiel 1 erfolgen. Die Viskositätszahlen, gemessen im Epprecht-Rheomat an dem vollständig mit Natronlauge neutralisierten Copolymerisat in 5 bzw. 1 gew.-%iger wäßriger Lösung bei 20 °C, betragen $4,2 \cdot 10^{-2}$ Pa·s bei D = $10000 \cdot s^{-1}$ und $610 \cdot 10^{-1}$ Pa·s bei D = $0,1 \cdot s^{-1}$.

**Beispiel 3**

Beispiel 1 wird wiederholt mit der Abänderung, daß das eingesetze Monomerengemisch aus 55 Gew.-% Ethylacrylat, 40 Gew.-% Methacrylsäure, 5 Gew.-% der monomeren Formel I-Urethanverbindung

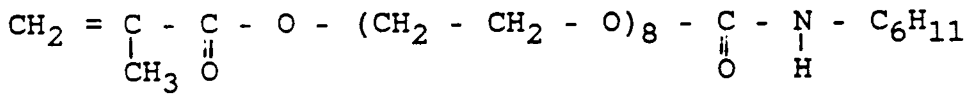

sowie zusätzlich 0,1 Gew.-%, bezogen auf die gesamte Monomerenmenge, n-Dodecylmerkaptan besteht. Der Feststoffgehalt der resultierenden Dispersion wird auf 30 Gew.-% eingestellt.

Die teilweise oder vollständige Neutralisation des Copolymerisatanteils sowie gegebenenfalls dessen Isolierung in trockener Form kann analog Beispiel 1 erfolgen. Die Viskositätszahlen, gemessen im Epprecht-Rheomat an dem vollständig mit Natronlauge neutralisierten Copolymerisat in 5 gew.-%iger wäßriger Lösung bei 20 °C, betragen $11 \cdot 10^{-2}$Pa•s bei D = $10000 \cdot s^{-1}$ und $18 \cdot 10^{-1}$ Pa•s bei D = $0{,}1 \cdot s^{-1}$.

**Beispiel 4**

Beispiel 1 wird wiederholt mit der Abänderung, daß das eingesetzte Monomerengemisch aus 72 Gew.-% Ethylacrylat, 18 Gew.-% Methacrylsäure, 10 Gew.-% der monomeren Formel I-Urethanverbindung

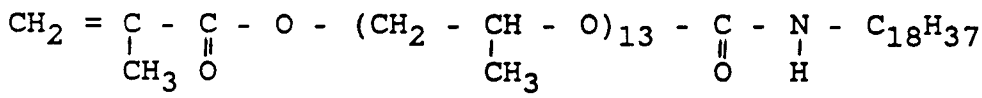

sowie zusätzlich 0,1 Gew.-%, bezogen auf die gesamte Monomerenmenge, N-Dodecylmerkaptan besteht. Der Feststoffgehalt der resultierenden Dispersion wird auf 30 Gew.-% eingestellt.

Die teilweise oder vollständige Neutralisation des Copolymerisatanteils sowie gegebenenfalls dessen Isolierung in trockener Form kann analog Beispiel 1 erfolgen. Die Viskositätszahlen, gemessen im Epprecht-Rheomat an dem vollständig mit Natronlauge neutralisierten Copolymerisat in 5 gew.-%iger wäßriger Lösung bei 20 °C, betragen $8{,}2 \cdot 10^{-2}$Pa•s bei D = $10000 \cdot s^{-1}$ und $4{,}5 \cdot 10^{-1}$ Pa•s bei D = $0{,}1 \cdot s^{-1}$.

**Beispiel 5**

Beispiel 1 wird wiederholt mit der Abänderung, daß das eingesetze Monomerengemisch aus 80 Gew.-% Ethylacrylat, 15 Gew.-% Methacrylsäure und 5 Gew.-% der monomeren Formel I-Urethanverbindung

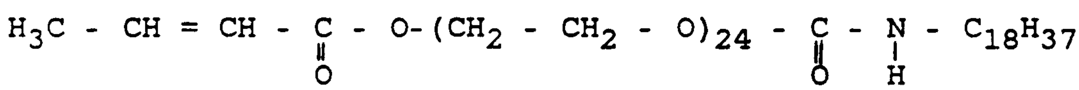

besteht.

Der Feststoffgehalt der resultierenden Dispersion wird auf 30 Gew.-% eingestellt.

Die teilweise oder vollständige Neutralisation des Copolymerisatanteils sowie gegebenenfalls dessen Isolierung in trockener Form kann analog Beispiel 1 erfolgen. Die Viskositätszahlen, gemessen im Epprecht-Rheomat an dem vollständig mit Natronlauge neutralisierten Copolymerisat in 1 gew.-%iger wäßriger Lösung bei 20 °C, betragen $9{,}1 \cdot 10^{-2}$Pa•s bei D = $10000 \cdot s^{-1}$ und $2{,}1 \cdot 10^{-1}$ Pa•s bei D = $0{,}1 \cdot s^{-1}$.

**Vergleichsbeispiel 1**

Beispiel 1 wird wiederholt mit der Abänderung, daß das eingesetzte Monomerengemisch aus 60 Gew.-% Ethylacrylat und 40 Gew.-% Methacrylsäure sowie zusätzlich 0,2 Gew.-%, bezogen auf die gesamte Monomerenmenge, n-Dodecylmerkaptan besteht.

Der Feststoffgehalt der resultierenden Dispersion wird auf 30 Gew.-% eingestellt.

Die teilweise oder vollständige Neutralisation des Copolymerisatanteils sowie gegebenenfalls dessen Isolierung in trockener Form kann analog Beispiel 1 erfolgen. Die Viskositätszahlen, gemessen im Epprecht-Rheomat an dem vollständig mit Natronlauge neutralisierten Copolymerisat in 5 gew.-%iger wäßriger Lösung bei 20 °C, betragen $5{,}2 \cdot 10^{-2}$Pa•s bei D = $10000 \cdot s^{-1}$ und $6{,}9 \cdot 10^{-2}$ Pa•s bei D = $0{,}1 \cdot s^{-1}$.

## Patentansprüche

## Patentansprüche für folgende Vertragsstaaten : BE, DE, FR, GB, IT

1. Copolymerisate auf der Basis von ethylenisch ungesättigten Monomeren, die Monomereinheiten aus grenzflächenaktiven Urethanderivaten mit ethylenisch ungesättigten Carboxy- oder Carbamidresten sowie Einheiten aus ethylenisch ungesättigten, salzbildungsfähigen Monomeren enthalten und durch radikalisch initiierte Lösungs-, Emulsions-, Suspensions-oder Perl-Copolymerisation hergestellt worden sind oder deren Lösungen oder wäßrigen Dispersionen oder deren Salze oder Lösungen bzw. Dispersionen der Salze, dadurch gekennzeichnet, daß die Copolymerisatpartikel, bezogen in Gew.-% auf die Gesamtmenge an Monomereinheiten in dem Copolymerisat, aufgebaut sind aus

   a) 25 bis 85 Gew-% ethylenisch ungesättigten hydrophoben Monomeren aus der Gruppe Vinylester von ($C_1$-$C_{18}$)-Monocarbonsäuren, (Meth-)Acrylester von ($C_1$-$C_{22}$)-Alkoholen, Vinylaromaten mit bis zu 18 C-Atomen, Vinylchlorid, Ethylen, (Meth-)Acrylnitril, Diester von Maleinsäure und/oder Fumarsäure mit ($C_1$-$C_{22}$)-Alkoholen, Vinylpyrrolidon, und

   b) 1 bis 50 Gew.-% ethylenisch ungesättigten, salzbildungsfähigen Monomeren mit funktionellen anionischen Resten aus der Gruppe -COOH, Sulfonsäuren bzw. Sulfonsäurederivate oder Phosphonsäuren bzw. Phosphonsäurederivate, Carbonsäuren aus der Gruppe ethylenisch ungesättigter ($C_3$-$C_5$)-Mono- oder Di-carbonsäuren und der Halbester der zweibasigen Carbonsäuren mit geradkettigen oder verzweigten ($C_1$-$C_8$)-Alkoholen, ferner Monomere aus der Gruppe Vinylsulfonsäure, (3-Sulfopropyl)methacrylsäureester, Acrylamidomethylpropansulfonsäure, Vinylphosphonsäure, Acrylamidomethylpropanphosphonsäure bzw. deren Salze, vorzugsweise Alkali- oder Ammoniumsalze,

   oder

   anstelle von anionischen Monomeren, ethylenisch ungesättigten, salzbildungsfähigen Monomeren mit funktionellen kationischen Resten aus der Gruppe $-NR^5R^6$, wobei $R^5$ und $R^6$ gleich oder verschieden sein können und H oder (C -$C_{18}$)-Alkyl bedeuten, oder $R^5$ und $R^6$ gemeinsam mit N gegebenenfalls einen fünf- bis siebengliedrigen heterocyclischen Ring bilden, oder ethylenisch ungesättigten ($C_3$-$C_{18}$)-aliphatischen primären Aminen oder sekundären Aminen mit einem ($C_1$-$C_{18}$)-Alkylrest oder tertiären Aminen mit zwei ($C_1$-$C_{18}$)-Alkylresten,

   und

   c) 0,1 bis 30 Gew.-% ethylenisch ungesättigten grenzflächenaktiven Urethanderivaten der Formel I,

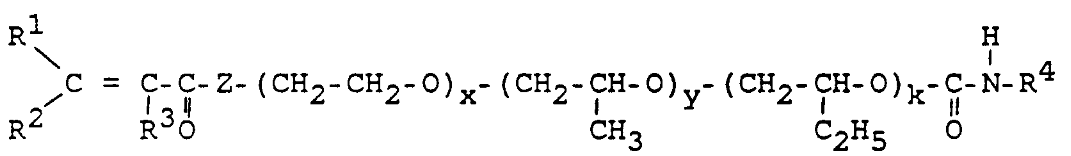

(I)

   worin die Reste $R^1$ bis $R^4$ und Z sowie die Zahlenindices x, y und k folgendes bedeuten:

   | | |
   |---|---|
   | $R^1$,$R^2$,$R^3$, | die gleich oder verschieden sein können, = H, $-CH_3$, -COOH, $-CH_2$-COOH, |
   | Z | = Sauerstoff oder NH, |
   | x,y,k, | die gleich oder verschieden sein können, = 0 bis 100 mit der Maßgabe, daß $x + y + k \geq 2$, |
   | $R^4$ | = gegebenenfalls substituiertes ($C_1$-$C_{30}$)-Alkyl, gegebenenfalls substituiertes ($C_6$-$C_{10}$)-Aryl, gegebenenfalls substituiertes ($C_7$-$C_{30}$)-Aralkyl, gegebenenfalls substituiertes ($C_5$-$C_8$)-Cycloalkyl, einen gegebenenfalls substituierten 5- bis 7-gliedrigen Heterocyclus, |

   und

   d) 0 bis 10 Gew.-% weiteren, von a) bis c) verschiedenen ethylenisch ungesättigten Monomeren mit funktionellen Resten aus der Gruppe -OH,

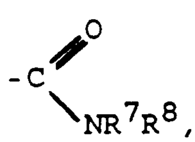

wobei $R^7$ und $R^8$ gleich oder verschieden sein können und für H, $(C_1-C_6)$-Alkyl, $(C_2-C_8)$-Alkoxyalkyl, $(C_5-C_7)$-Cycloalkyl oder $(C_6-C_{18})$-Aralkyl stehen, oder $R^7$ und $R^8$ gemeinsam mit N gegebenenfalls einen fünf- bis siebengliedrigen heterocyclischen Ring bilden, vorzugsweise ethylenisch ungesättigten Hydroxyalkylestern oder Polyalkylenoxidestern der (Meth-)Acrylsäure, insbesondere mit 2 bis 50 Ethylenoxideinheiten und/oder 2 bis 50 Propylenoxideinheiten, wobei die endständigen OH-Gruppen der Ester- bzw. der Polyalkylenglykoläther-reste auch veräthert oder verestert sein können, ethylenisch ungesättigten Amiden,

und

e) 0 bis 5 Gew.-% ethylenisch ungesättigten Carbonylverbindungen, vorzugsweise aus der Gruppe Vinylmethylketon, Acrolein, Crotonaldehyd, Allylacetoacetat, Acetoacetoxyethyl(meth-)acrylat,

und

f) 0 bis 5 Gew.-% mehrfach ethylenisch ungesättigten bzw. mehrfachfunktionellen und zur Vernetzung befähigten Monomeren, vorzugsweise aus der Gruppe Divinylbenzol, Diallylphthalat, Butandioldiacrylat, Triethylenglykoldimethacrylat, Allylmethacrylat, Bisphenol-A-diethylenglykoldimethacrylat, Triallylcyanurat, Methylen-bis-methacrylamid,

und

g) 0 bis 5 Gew.-% Molekulargewichtsreglern aus der Gruppe Dodecylmerkaptan, Tetrachlorkohlenstoff, $\alpha$-Methylstyrol, Toluol, Bromtrichlormethan, Tetrakismerkaptoacetylpentaerythrit, Thioglykolsäure,

und bei Vorliegen wäßriger Dispersionen bzw. der daraus isolierten Copolymerisate diese außerdem

h) 0,1 bis 10 Gew.-%, bezogen auf die Gesamtmenge aller Monomereinheiten in dem Copolymerisat, Emulgatoren und/oder gegebenenfalls Schutzkolloide, vorzugsweise aus der Gruppe der anionischen oder kationischen oder zwitterionischen und/oder insbesondere der nichtionogenen Tenside und/oder Schutzkolloide, enthalten.

**2.** Copolymerisate nach Anspruch 1, dadurch gekennzeichnet, daß sie in teilweise oder vollständig neutralisierter wasserlöslicher oder in Wasser kolloidal dispergierbarer Form vorliegen.

**3.** Verwendung der Copolymerisate nach Ansprüchen 1 oder 2, zum Verdicken von wäßrigen Systemen, insbesondere wäßrigen Dispersionen.

**4.** Verwendung der Copolymerisate nach Ansprüchen 1 oder 2, zum Verdicken von Dispersionsfarben, Dispersionsglanzlacken, Textildruckpasten, Papierdruckpasten, bioziden Wirkstoffzübereitungen, Flüssigdüngemitteln, Emulsionsreinigern, Abbeizpasten, Enteisungsmitteln und kosmetischen Zubereitungen.

**5.** Verwendung der Copolymerisate nach Ansprüchen 1 oder 2, zum Verdicken von wäßrigen Systemen, insbesondere wäßrigen Dispersionen, indem man die Copolymerisate in ihrer salzbildungsfähigen, nicht neutralisierten Form als Lösungen oder Dispersionen in der erforderlichen Menge mit dem zu verdickenden wäßrigen System, insbesondere der zu verdickenden Dispersion, vermischt und das resultierende Gemisch anschließend, bei anionischen Copolymerisaten durch Zusatz von Basen teilweise oder vollständig neutralisiert oder schwach alkalisch einstellt, oder bei kationischen Copolymerisaten durch Zusatz von Säuren teilweise oder vollständig neutralisiert oder schwach sauer einstellt, so daß die Copolymerisate als in Wasser lösliche oder kolloidal dispergierbare Copolymerisatsalze vorliegen.

**6.** Verwendung der Copolymerisate nach Ansprüchen 1 oder 2 als Schlichtemittel in der Textilindustrie.

**7.** Verwendung der Copolymerisate als Verdicker nach einem oder mehreren der Ansprüche 1 bis 5 in Einsatzmengen von 0,01 bis 5 Gew.-% Copolymerisat, bezogen auf das zu verdickende wäßrige System.

**8.** Verfahren zur Herstellung von Copolymerisaten durch radikalisch initiierte Emulsions- oder Lösungscopolymerisation ethylenisch ungesättigter copolymerisationsfähiger Monomerer sowie gegebenenfalls nachfolgender teilweiser oder vollständiger Neutralisation der salzbildungsfähigen Gruppen und gegebenenfalls Isolierung der Copolymerisate in getrockneter Form, dadurch gekennzeichnet, daß man als Comonomere

a) 25 bis 85 Gew-% ethylenisch ungesättigte, hydrophobe Monomere aus der Gruppe Vinylester von $(C_1-C_{18})$-Monocarbonsäuren, (Meth-)Acrylester von $(C_1-C_{22})$-Alkoholen, Vinylaromaten mit bis zu 18

C-Atomen, Vinylchlorid, Ethylen, (Meth-)Acrylnitril, Diester von Maleinsäure und/oder Fumarsäure mit ($C_1$-$C_{22}$)-Alkoholen, Vinylpyrrolidon,

und

b) 1 bis 50 Gew.-% ethylenisch ungesättigte, salzbildungsfähige Monomere mit funktionellen anionischen Resten aus der Gruppe -COOH, Sulfonsäuren bzw. Sulfonsäurederivate oder Phosphonsäuren bzw. Phosphonsäurederivate, Carbonsäuren aus der Gruppe ethylenisch ungesättigter ($C_3$-$C_5$)-Mono- oder Di-carbonsäuren und der Halbester der zweibasigen Carbonsäuren mit geradkettigen oder verzweigten ($C_1$-$C_8$)-Alkoholen, ferner Monomere aus der Gruppe Vinylsulfonsäure, (3-Sulfopropyl)methacrylsäureester, Acrylamidomethylpropansulfonsäure, Vinylphosphonsäure, Acrylamidomethylpropanphosphonsäure bzw. deren Salze, vorzugsweise Alkali- oder Ammoniumsalze,

oder

anstelle von anionischen Monomeren, ethylenisch ungesättigte, salzbildungsfähige Monomere mit funktionellen kationischen Resten aus der Gruppe $-NR^5R^6$, wobei $R^5$ und $R^6$ gleich oder verschieden sein können und H oder ($C_1$-$C_{18}$)-Alkyl bedeuten, oder $R^5$ und $R^6$ gemeinsam mit N gegebenenfalls einen fünf- bis siebengliedrigen heterocyclischen Ring bilden, oder ethylenisch ungesättigte ($C_3$-$C_{18}$)-aliphatische primäre Amine oder sekundäre Amine mit einem ($C_1$-$C_{18}$)-Alkylrest oder tertiäre Amine mit zwei ($C_1$-$C_{18}$)-Alkylresten,

und

c) 0,1 bis 30 Gew.-% ethylenisch ungesättigte grenzflächenaktive Urethanderivate der Formel I,

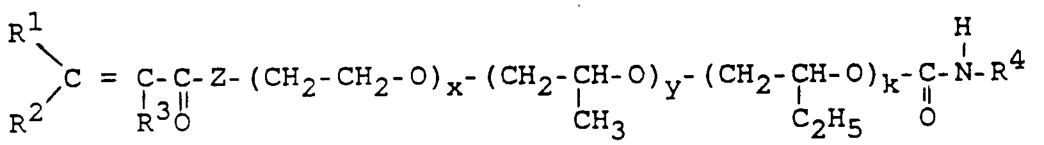 (I)

worin die Reste $R^1$ bis $R^4$ und Z sowie die Zahlenindices x, y und k folgendes bedeuten:

| | |
|---|---|
| $R^1,R^2,R^3$, | die gleich oder verschieden sein können, = H, $-CH_3$, -COOH, $-CH_2$-COOH, |
| Z | = Sauerstoff oder NH, |
| x,y,k, | die gleich oder verschieden sein können, = 0 bis 100 mit der Maßgabe, daß $x + y + k \geqq 2$, |
| $R^4$ | = gegebenenfalls substituiertes ($C_1$-$C_{30}$)-Alkyl, gegebenenfalls substituiertes ($C_6$-$C_{10}$)-Aryl, gegebenenfalls substituiertes ($C_7$-$C_{30}$)-Aralkyl, gegebenenfalls substituiertes ($C_5$-$C_8$)-Cycloalkyl, einen gegebenenfalls substituierten 5- bis 7-gliedrigen Heterocyclus, |

und

d) 0 bis 10 Gew.-% weitere, von a) bis c) verschiedene ethylenisch ungesättigte Monomere mit funktionellen Resten aus der Gruppe -OH,

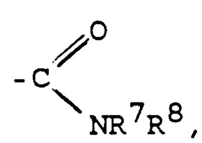

wobei $R^7$ und $R^8$ gleich oder verschieden sein können und für H, ($C_1$-$C_6$)-Alkyl, ($C_2$-$C_8$)-Alkoxyalkyl, ($C_5$-$C_7$)-Cycloalkyl oder ($C_6$-$C_{18}$)-Aralkyl stehen, oder $R^7$ und $R^8$ gemeinsam mit N gegebenenfalls einen fünf- bis siebengliedrigen heterocyclischen Ring bilden, vorzugsweise ethylenisch ungesättigte Hydroxyalkylester oder Polyalkylenoxidester der (Meth-)Acrylsäure, insbesondere mit 2 bis 50 Ethylenoxideinheiten und/oder 2 bis 50 Propylenoxideinheiten, wobei die endständigen OH-Gruppen der Ester- bzw. der Polyalkylenglykoläther-reste auch veräthert oder verestert sein können, ethylenisch ungesättigte Amide,

und

e) 0 bis 5 Gew.-% ethylenisch ungesättigte Carbonylverbindungen, vorzugsweise aus der Gruppe Vinylmethylketon, Acrolein, Crotonaldehyd, Allylacetoacetat, Acetoacetoxyethyl(meth-)acrylat,

und

f) 0 bis 5 Gew.-% mehrfach ethylenisch ungesättigte bzw. mehrfachfunktionelle und zur Vernetzung befähigte Monomere, vorzugsweise aus der Gruppe Divinylbenzol, Diallylphthalat, Butandioldiacrylat, Triethylenglykoldimethacrylat, Allylmethacrylat, Bisphenol-A-diethylenglykoldimethacrylat, Triallylcyanurat, Methylen-bis-methacrylamid,

und

g) 0 bis 5 Gew.-% Molekulargewichtsregler aus der Gruppe Dodecylmerkaptan, Tetrachlorkohlenstoff, $\alpha$-Methylstyrol, Toluol, Bromtrichlormethan, Tetrakismerkaptoacetylpentaerythrit, Thioglykolsäure,

copolymerisiert und bei der Emulsionscopolymerisation außerdem

h) 0,1 bis 10 Gew.-%, bezogen auf die Gesamtmenge aller Monomereinheiten in dem Copolymerisat, Emulgatoren und/oder gegebenenfalls Schutzkolloide, vorzugsweise aus der Gruppe der anionischen oder kationischen oder zwitterionischen und/oder insbesondere der nichtionogenen Tenside und/oder Schutzkolloide, zusetzt,

das resultierende Copolymerisat gegebenenfalls durch teilweise oder vollständige Neutralisation, und zwar bei anionischen Copolymerisaten durch Zusatz von Basen und bei kationischen Copolymerisaten durch Zusatz von Säuren in ein wasserlösliches oder in Wasser kolloidal dispergierbares Copolymerisatsalz überführt und gegebenenfalls das copolymere Produkt durch übliche Eliminierung des Lösungs- oder Dispergiermittels in fester Form gewinnt.

**9.** Verfahren nach Anspruch 8, dadurch gekennzeichnet, daß man bei der Emulsionscopolymerisation die grenzflächenaktive comonomere Komponente c) in der Wasserphase vorlegt.

**Patentansprüche für folgenden Vertragsstaat : ES**

**1.** Verfahren zur Herstellung von Copolymerisaten auf der Basis von ethylenisch ungesättigten Monomeren, die Monomereinheiten aus grenzflächenaktiven Urethanderivaten mit ethylenisch ungesättigten Carboxy- oder Carbamidresten sowie Einheiten aus ethylenisch ungesättigten salzbildungsfähigen Monomeren enthalten durch radikalisch initiierte Emulsions-, Suspensions-, Perl- oder Lösungscopolymerisation ethylenisch ungesättigter copolymerisationsfähiger Monomerer sowie gegebenenfalls nachfolgender teilweiser oder vollständiger Neutralisation der salzbildungsfähigen Gruppen und gegebenenfalls Isolierung der Copolymerisate in getrockneter Form, dadurch gekennzeichnet, daß man als Comonomere

a) 25 bis 85 Gew-% ethylenisch ungesättigte, hydrophobe Monomere aus der Gruppe Vinylester von ($C_1$-$C_{18}$)-Monocarbonsäuren, (Meth-)Acrylester von ($C_1$-$C_{22}$)-Alkoholen, Vinylaromaten mit bis zu 18 °C-Atomen, Vinylchlorid, Ethylen, (Meth-)Acrylnitril, Diester von Maleinsäure und/oder Fumarsäure mit ($C_1$-$C_{22}$)-Alkoholen, Vinylpyrrolidon, und

b) 1 bis 50 Gew.-% ethylenisch ungesättigte, salzbildungsfähige Monomere mit funktionellen anionischen Resten aus der Gruppe -COOH, Sulfonsäuren bzw. Sulfonsäurederivate oder Phosphonsäuren bzw. Phosphonsäurederivate, Carbonsäuren aus der Gruppe ethylenisch ungesättigter ($C_3$-$C_5$)-Mono- oder Di-carbonsäuren und der Halbester der zweibasigen Carbonsäuren mit geradkettigen oder verzweigten ($C_1$-$C_8$)-Alkoholen, ferner Monomere aus der Gruppe Vinylsulfonsäure, (3-Sulfopropyl)methacrylsäureester, Acrylamidomethylpropansulfonsäure, Vinylphosphonsäure, Acrylamidomethylpropanphosphonsäure bzw. deren Salze, vorzugsweise Alkali- oder Ammoniumsalze,

oder

anstelle von anionischen Monomeren, ethylenisch ungesättigte, salzbildungsfähige Monomere mit funktionellen kationischen Resten aus der Gruppe $-NR^5R^6$, wobei $R^5$ und $R^6$ gleich oder verschieden sein können und H oder ($C_1$-$C_{18}$)-Alkyl bedeuten, oder $R^5$ und $R^6$ gemeinsam mit N gegebenenfalls einen fünf- bis siebengliedrigen heterocyclischen Ring bilden, oder ethylenisch ungesättigte ($C_3$-$C_{18}$)-aliphatische primäre Amine oder sekundäre Amine mit einem ($C_1$-$C_{18}$)-Alkylrest oder tertiäre Amine mit zwei ($C_1$-$C_{18}$)-Alkylresten,

und

c) 0,1 bis 30 Gew.-% ethylenisch ungesättigte grenzflächenaktive Urethanderivate der Formel I,

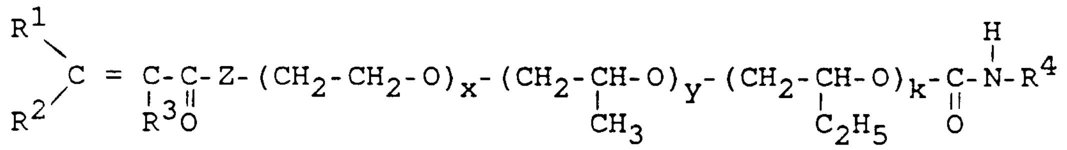

(I)

worin die Reste $R^1$ bis $R^4$ und Z sowie die Zahlenindices x, y und k folgendes bedeuten:

| | |
|---|---|
| $R^1$,$R^2$,$R^3$, | die gleich oder verschieden sein können, |
| | = H, $-CH_3$, -COOH, $-CH_2$-COOH, |
| Z | = Sauerstoff oder NH, |
| x,y,k, | die gleich oder verschieden sein können, |
| | = 0 bis 100 mit der Maßgabe, daß $x + y + k \geqq 2$, |
| $R^4$ | = gegebenenfalls substituiertes ($C_1$-$C_{30}$)-Alkyl, gegebenenfalls substituiertes ($C_6$-$C_{10}$)-Aryl, gegebenenfalls substituiertes ($C_7$-$C_{30}$)-Aralkyl, gegebenenfalls substituiertes ($C_5$-$C_8$)-Cycloalkyl, einen gegebenenfalls substituierten 5- bis 7-gliedrigen Heterocyclus, |

und

d) 0 bis 10 Gew.-% weitere, von a) bis c) verschiedene ethylenisch ungesättigte Monomere mit funktionellen Resten aus der Gruppe -OH,

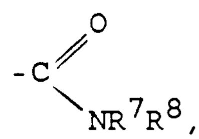

wobei $R^7$ und $R^8$ gleich oder verschieden sein können und für H, ($C_1$-$C_6$)-Alkyl, ($C_2$-$C_8$)-Alkoxyalkyl, ($C_5$-$C_7$)-Cycloalkyl oder ($C_6$-$C_{18}$)-Aralkyl stehen, oder $R^7$ und $R^8$ gemeinsam mit N gegebenenfalls einen fünf- bis siebengliedrigen heterocyclischen Ring bilden, vorzugsweise ethylenisch ungesättigte Hydroxyalkylester oder Polyalkylenoxidester der (Meth-)Acrylsäure, insbesondere mit 2 bis 50 Ethylenoxideinheiten und/oder 2 bis 50 Propylenoxideinheiten, wobei die endständigen OH-Gruppen der Ester- bzw. der Polyalkylenglykolätherreste auch veräthert oder verestert sein können, ethylenisch ungesättigte Amide,

und

e) 0 bis 5 Gew.-% ethylenisch ungesättigte Carbonylverbindungen, vorzugsweise aus der Gruppe Vinylmethylketon, Acrolein, Crotonaldehyd, Allylacetoacetat, Acetoacetoxyethyl(meth-)acrylat,

und

f) 0 bis 5 Gew.-% mehrfach ethylenisch ungesättigte bzw. mehrfachfunktionelle und zur Vernetzung befähigte Monomere, vorzugsweise aus der Gruppe Divinylbenzol, Diallylphthalat, Butandioldiacrylat, Triethylenglykoldimethacrylat, Allylmethacrylat, Bisphenol-A-diethylenglykoldimethacrylat, Triallylcyanurat, Methylen-bis-methacrylamid,

und

g) 0 bis 5 Gew.-% Molekulargewichtsregler aus der Gruppe Dodecylmerkaptan, Tetrachlorkohlenstoff, $\alpha$-Methylstyrol, Toluol, Bromtrichlormethan, Tetrakismerkaptoacetylpentaerythrit, Thioglykolsäure,

nach üblichen Methoden radikalisch copolymerisiert und bei der Emulsionscopolymerisation außerdem

h) 0,1 bis 10 Gew.-%, bezogen auf die Gesamtmenge aller Monomereinheiten in dem Copolymerisat, Emulgatoren und/oder gegebenenfalls Schutzkolloide, vorzugsweise aus der Gruppe der anionischen oder kationischen oder zwitterionischen und/oder insbesondere der nichtionogenen Tenside und/oder Schutzkolloide, zusetzt,

das resultierende Copolymerisat gegebenenfalls durch teilweise oder vollständige Neutralisation, und zwar bei anionischen Copolymerisaten durch Zusatz von Basen und bei kationischen Copolymerisaten durch Zusatz von Säuren in ein wasserlösliches oder in Wasser kolloidal dispergierbares Copolymerisatsalz überführt und gegebenenfalls das copolymere Produkt durch übliche Eliminierung des Lösungs- oder Dispergiermittels in fester Form gewinnt.

**2.** Verfahren nach Anspruch 1, dadurch gekennzeichnet, daß man bei der Emulsionscopolymerisation die grenzflächenaktive comonomere Komponente c) in der Wasserphase vorlegt.

**3.** Verfahren nach Ansprüchen 1 oder 2, dadurch gekennzeichnet, daß die Copolymerisate in teilweise oder vollständig neutralisierter wasserlöslicher oder in Wasser kolloidal dispergierbarer Form erhalten werden.

**4.** Verwendung der Copolymerisate, hergestellt nach Ansprüchen 1, 2 oder 3, zum Verdicken von wäßrigen Systemen, insbesondere wäßrigen Dispersionen.

**5.** Verwendung der Copolymerisate, hergestellt nach Ansprüchen 1, 2 oder 3, zum Verdicken von Dispersionsfarben, Dispersionsglanzlacken, Textildruckpasten, Papierdruckpasten, bioziden Wirkstoffzübereitungen, Flüssigdüngemitteln, Emulsionsreinigern, Abbeizpasten, Enteisungsmitteln und kosmetischen Zübereitungen.

**6.** Verwendung der Copoloymerisate, hergestellt nach Ansprüchen 1 oder 2, zum Verdicken von wäßrigen Systemen, insbesondere wäßrigen Dispersionen, indem man die Copolymerisate in ihrer salzbildungsfähigen, nicht neutralisierten Form als Lösungen oder Dispersionen in der erforderlichen Menge mit dem zu verdickenden wäßrigen System, insbesondere der zu verdickenden Dispersion, vermischt und das resultierende Gemisch anschließend, bei anionischen Copolymerisaten durch Zusatz von Basen teilweise oder vollständig neutralisiert oder schwach alkalisch einstellt, oder bei kationischen Copolymerisaten durch Zusatz von Säuren teilweise oder vollständig neutralisiert oder schwach sauer einstellt, so daß die Copolymerisate als in Wasser lösliche oder kolloidal dispergierbare Copolymerisatsalze vorliegen.

**7.** Verwendung der Copolymerisate, hergestellt nach Ansprüchen 1, 2 oder 3, als Schlichtemittel in der Textilindustrie.

**8.** Verwendung der Copolymerisate, hergestellt nach Ansprüchen 1, 2 oder 3, als Verdicker in Einsatzmengen von 0,01 bis 5 Gew.-% Copolymerisat, bezogen auf das zu verdickende wäßrige System.

**Claims**
**Claims for the following Contracting States : BE, DE, FR, GB, IT**

**1.** A copolymer based on ethylenically unsaturated monomers comprising monomer units of surface-active urethane derivatives with ethylenically unsaturated carboxyl or carbamido radicals and units of ethylenically unsaturated monomers capable of salt formation and having been prepared by free-radical-initiated copolymerization in solution, emulsion, suspension or by bead copolymerization or solutions or aqueous dispersions thereof or the salts thereof or solutions or dispersions of the salts, wherein the copolymer particles, relative to the total amount of monomer units in the copolymer in % by weight, have preferably been synthesized from

a) 25 to 85% by weight of ethylenically unsaturated hydrophobic monomers from the group comprising vinyl esters of ($C_1$-$C_{18}$)-monocarboxylic acids, (meth)acrylic esters of ($C_1$-$C_{22}$)-alcohols, vinyl aromatics having up to 18 carbon atoms, vinyl chloride, ethylene, (meth)acrylonitrile, diesters of maleic acid and/or fumaric acid with ($C_1$-$C_{22}$)-alcohols, vinylpyrrolidone, and

b) 1 to 50% by weight of ethylenically unsaturated monomers capable of salt formation and containing functional anionic radicals from the group comprising -COOH, sulfonic acids or sulfonic acid derivatives or phosphonic acids or phosphonic acid derivatives, carboxylic acids from the group comprising ethylenically unsaturated ($C_3$-$C_5$)-mono- or dicarboxylic acids, and monoesters of dibasic carboxylic acids with straight-chain or branched ($C_1$-$C_8$)-alcohols, furthermore monomers from the group comprising vinylsulfonic acid, (3-sulfopropyl)methacrylic esters, acrylamidomethylpropanesulfonic acid, vinylphosphonic acid, acrylamidomethylpropanephosphonic acid or salts thereof, preferably alkali metal salts or ammonium salts,

or

instead of anionic monomers, ethylenically unsaturated monomers capable of salt formation and containing functional cationic radicals from the group comprising $-NR^5R^6$, where $R^5$ and $R^6$ can be identical or different and be H or ($C_1$-$C_{18}$)-alkyl, or $R^5$ and $R^6$ together with N can, if desired, form a five- to seven-membered heterocyclic ring, or ethylenically unsaturated ($C_3$-$C_{18}$)-aliphatic primary

amines or secondary amines containing a ($C_1$-$C_{18}$)-alkyl radical or tertiary amines containing two ($C_1$-$C_{18}$)-alkyl radicals,
and
c) 0.1 to 30% by weight of ethylenically unsaturated surface-active urethane derivatives of the formula I,

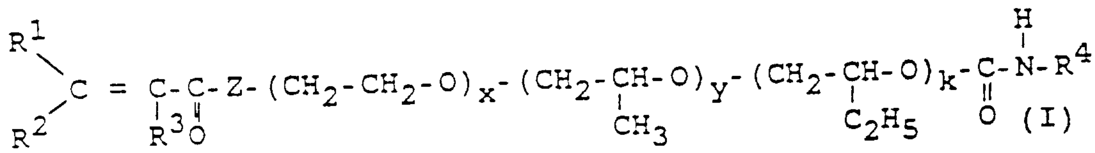
(I)

in which the radicals $R^1$ to $R^4$ and Z and the numbered indices x, y and k have the following meanings:

$R^1$, $R^2$, $R^3$, which can be identical or different, are H, $-CH_3$, -COOH, $-CH_2$-COOH,
Z is oxygen or NH,
x, y, k, which can be identical or different, are 0 to 100, with the proviso that $x+y+k \geq 2$,
$R^4$ is substituted or unsubstituted ($C_1$-$C_{30}$)-alkyl, substituted or unsubstituted ($C_6$-$C_{10}$)-aryl, substituted or unsubstituted ($C_7$-$C_{30}$)-aralkyl, substituted or unsubstituted ($C_5$-$C_8$)-cycloalkyl, a substituted or unsubstituted 5- to 7-membered heterocycle,

and
d) 0 to 10% by weight of further ethylenically unsaturated monomers different from a) to c) and having functional radicals from the group comprising -OH,

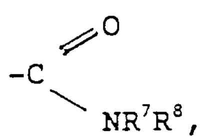

where $R^7$ and $R^8$ can be identical or different and are H, ($C_1$-$C_6$)-alkyl, ($C_2$-$C_8$)-alkoxyalkyl, ($C_5$-$C_7$)-cycloalkyl or ($C_6$-$C_{18}$)-aralkyl, or $R^7$ and $R^8$ together with N form, if desired, a five- to seven-membered heterocyclic ring, preferably ethylenically unsaturated hydroxyalkyl esters or polyalkyleneoxide esters of (meth)acrylic acid, in particular containing 2 to 50 ethylene oxide units and/or 2 to 50 propylene oxide units, in which the terminal OH groups of the ester or polyalkylene glycol ether radicals can also be etherified or esterified, ethylenically unsaturated amides,
and
e) 0 to 5% by weight of ethylenically unsaturated carbonyl compounds, preferably from the group comprising vinyl methyl ketone, acrolein, crotonaldehyde, allyl acetoacetate, acetoacetoxyethyl (meth)acrylate,
and
f) 0 to 5% by weight of ethylenically polyunsaturated or polyfunctional monomers capable of crosslinking, preferably from the group comprising divinylbenzene, diallyl phthalate, butanediol diacrylate, triethylene glycol dimethacrylate, allyl methacrylate, bisphenol A diethylene glycol dimethacrylate, triallyl cyanurate, methylene-bis(meth)acrylamide,
and
g) 0 to 5% by weight of molecular weight regulators from the group comprising dodecylmercaptan, carbon tetrachloride, $\alpha$-methylstyrene, toluene, bromotrichloromethane, tetrakis(mercaptoacetyl)-pentaerythritol, thioglycolic acid,

and when aqueous dispersions or the copolymers isolated therefrom are present, they furthermore contain

h) 0.1 to 10% by weight, relative to the total amount of all monomer units in the copolymer, of emulsifiers and/or if desired, protective colloids, preferably from the group comprising anionic or cationic or zwitter ionic and/or in particular nonionic surfactants and/or protective colloids.

**2.** Copolymers as claimed in claim 1, which are present in partially or completely neutralized water-soluble or colloid-water-dispersible form.

**3.** Use of the copolymers as claimed in claims 1 or 2 for the thickening of aqueous systems, in particular aqueous dispersions.

**4.** Use of the copolymers as claimed in claims 1 or 2 for the thickening of emulsion paints, gloss emulsion paints, textile printing pastes, paper printing pastes, preparations of biocides, liquid fertilizers, emulsion cleaners, pickling pastes, de-icing agents and cosmetic preparations.

**5.** Use of the copolymers as claimed in claims 1 or 2 for the thickening of aqueous systems, in particular aqueous dispersions, by mixing the copolymers in their unneutralized form capable of salt formation as solutions or dispersions in the required amount with the aqueous system to be thickened, in particular with the dispersion to be thickened, and then partially or completely neutralizing the resulting mixture by addition of bases in the case of anionic copolymers or making it weakly alkaline, or partially or completely neutralizing by addition of acids in the case of cationic copolymers or making it weakly acidic, so that the copolymers are present as water-soluble or colloid-dispersible copolymer salts.

**6.** Use of the copolymers as claimed in claims 1 or 2 as sizing material in the textile industry.

**7.** Use of the copolymers as thickener as claimed in one or more of claims 1 to 5 in amounts of 0.01 to 5% by weight of copolymer, relative to the aqueous system to be thickened.

**8.** A process for the preparation of copolymers by free-radical-initiated emulsion copolymerization or solution copolymerization of ethylenically unsaturated copolymerizable monomers and, if desired, subsequent partial or complete neutralization of the groups capable of salt formation and, if desired, isolation of the copolymers in dried form, which comprises copolymerizing as comonomers

a) 25 to 85% by weight of ethylenically unsaturated hydrophobic monomers from the group comprising vinyl esters of ($C_1$-$C_{18}$)-monocarboxylic acids, (meth)acrylic esters of ($C_1$-$C_{22}$)-alcohols, vinyl aromatics having up to 18 carbon atoms, vinyl chloride, ethylene, (meth)acrylonitrile, diesters of maleic acid and/or fumaric acid with ($C_1$-$C_{22}$)-alcohols, vinylpyrrolidone, and

b) 1 to 50% by weight of ethylenically unsaturated monomers capable of salt formation and containing functional anionic radicals from the group comprising -COOH, sulfonic acids or sulfonic acid derivatives or phosphonic acids or phosphonic acid derivatives, carboxylic acids from the group comprising ethylenically unsaturated ($C_3$-$C_5$)-mono- or dicarboxylic acids, and monoesters of dibasic carboxylic acids with straight-chain or branched ($C_1$-$C_8$)-alcohols, furthermore monomers from the group comprising vinylsulfonic acid, (3-sulfopropyl)methacrylic esters, acrylamidomethylpropanesulfonic acid, vinylphosphonic acid, acrylamidomethylpropanephosphonic acid or salts thereof, preferably alkali metal salts or ammonium salts,

or

instead of anionic monomers, ethylenically unsaturated monomers capable of salt formation and containing functional cationic radicals from the group comprising $-NR^5R^6$, where $R^5$ and $R^6$ can be identical or different and be H or ($C_1$-$C_{18}$)-alkyl, or $R^5$ and $R^6$ together with N can, if desired, form a five- to seven-membered heterocyclic ring, or ethylenically unsaturated ($C_3$-$C_{18}$)-aliphatic primary amines or secondary amines containing a ($C_1$-$C_{18}$)-alkyl radical or tertiary amines containing two ($C_1$-$C_{18}$)-alkyl radicals,

and

c) 0.1 to 30% by weight of ethylenically unsaturated surface-active urethane derivatives of the formula I,

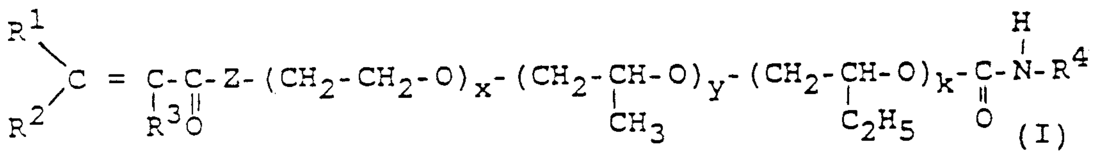

(I)

in which the radicals $R^1$ to $R^4$ and Z and the numbered indices x, y and k have the following meanings:

| | |
|---|---|
| $R^1$, $R^2$, $R^3$, | which can be identical or different, are H, $-CH_3$, -COOH, $-CH_2$-COOH, |
| Z | is oxygen or NH, |

x, y, k, which can be identical or different, are 0 to 100, with the proviso that $x + y + k \geqq 2$,
$R^4$ is substituted or unsubstituted ($C_1$-$C_{30}$)-alkyl, substituted or unsubstituted ($C_6$-$C_{10}$)-aryl, substituted or unsubstituted ($C_7$-$C_{30}$)-aralkyl, substituted or unsubstituted ($C_5$-$C_8$)-cycloalkyl, a substituted or unsubstituted 5- to 7-membered heterocycle,

and

d) 0 to 10% by weight of further ethylenically unsaturated monomers different from a) to c) and having functional radicals from the group comprising -OH,

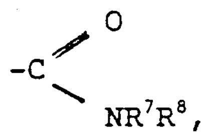

where $R^7$ and $R^8$ can be identical or different and are H, ($C_1$-$C_6$)-alkyl, ($C_2$-$C_8$)-alkoxyalkyl, ($C_5$-$C_7$)-cycloalkyl or ($C_6$-$C_{18}$)-aralkyl, or $R^7$ and $R^8$ together with N form, if desired, a five- to seven-membered heterocyclic ring, preferably ethylenically unsaturated hydroxyalkyl esters or polyalkyleneoxide esters of (meth)acrylic acid, in particular containing 2 to 50 ethylene oxide units and/or 2 to 50 propylene oxide units, in which the terminal OH groups of the ester or polyalkylene glycol ether radicals can also be etherified or esterified, ethylenically unsaturated amides,

and

e) 0 to 5% by weight of ethylenically unsaturated carbonyl compounds, preferably from the group comprising vinyl methyl ketone, acrolein, crotonaldehyde, allyl acetoacetate, acetoacetoxyethyl (meth)acrylate,

and

f) 0 to 5% by weight of ethylenically polyunsaturated or polyfunctional monomers capable of crosslinking, preferably from the group comprising divinylbenzene, diallyl phthalate, butanediol diacrylate, triethylene glycol dimethacrylate, allyl methacrylate, bisphenol A diethylene glycol dimethacrylate, triallyl cyanurate, methylene-bis(meth)acrylamide,

and

g) 0 to 5% by weight of molecular weight regulators from the group comprising dodecylmercaptan, carbon tetrachloride, $\alpha$-methylstyrene, toluene, bromotrichloromethane, tetrakis(mercaptoacetyl)-pentaerythritol, thioglycolic acid,

and furthermore in the case of emulsion copolymerization adding

h) 0.1 to 10% by weight, relative to the total amount of all monomer units in the copolymer, of emulsifiers and/or if desired, protective colloids, preferably from the group comprising anionic or cationic or zwitter ionic and/or in particular nonionic surfactants and/or protective colloids,

converting the resulting copolymer, if appropriate by partial or complete neutralization, by addition of bases in the case of anionic copolymers and by addition of acids in the case of cationic copolymers into a water-soluble or colloid-water-dispersible copolymer salt and, if desired, obtaining the copolymer product in solid form by eliminating the solvent or dispersant in a conventional manner.

9. The process as claimed in claim 8, wherein during the emulsion copolymerization the surface-active comonomer component c) is initially introduced into the water phase.

**Claims for the following Contracting State : ES**

1. A process for the preparation of copolymers based on ethylenically unsaturated monomers comprising monomer units of surface-active urethane derivatives with ethylenically unsaturated carboxyl or carbamido radicals and units of ethylenically unsaturated monomers capable of salt formation, by free-radical-initiated copolymerization in solution, emulsion, suspension or by bead or solution copolymerization of ethylenically unsaturated copolymerizable monomers and, if desired, subsequent partial or complete neutralization of the groups capable of salt formation and, if desired, isolation of the copolymers in dried form, which comprises copolymerizing as comonomers

a) 25 to 85% by weight of ethylenically unsaturated hydrophobic monomers from the group comprising vinyl esters of ($C_1$-$C_{18}$)-monocarboxylic acids, (meth)acrylic esters of ($C_1$-$C_{22}$)-alcohols, vinyl aromatics having up to 18 carbon atoms, vinyl chloride, ethylene, (meth)acrylonitrile, diesters of maleic acid and/or fumaric acid with ($C_1$-$C_{22}$)-alcohols, vinylpyrrolidone, and

b) 1 to 50% by weight of ethylenically unsaturated monomers capable of salt formation and containing functional anionic radicals from the group comprising -COOH, sulfonic acids or sulfonic acid derivatives or phosphonic acids or phosphonic acid derivatives, carboxylic acids from the group comprising ethylenically unsaturated ($C_3$-$C_5$)-mono- or dicarboxylic acids, and monoesters of dibasic carboxylic acids with straight-chain or branched ($C_1$-$C_8$)-alcohols, furthermore monomers from the group comprising vinylsulfonic acid, (3-sulfopropyl)methacrylic esters, acrylamidomethylpropanesulfonic acid, vinylphosphonic acid, acrylamidomethylpropanephosphonic acid or salts thereof, preferably alkali metal salts or ammonium salts,

or

instead of anionic monomers, ethylenically unsaturated monomers capable of salt formation and containing functional cationic radicals from the group comprising $-NR^5R^6$, where $R^5$ and $R^6$ can be identical or different and be H or ($C_1$-$C_{18}$)-alkyl, or $R^5$ and $R^6$ together with N can, if desired, form a five- to seven-membered heterocyclic ring, or ethylenically unsaturated ($C_3$-$C_{18}$)-aliphatic primary amines or secondary amines containing a ($C_1$-$C_{18}$)-alkyl radical or tertiary amines containing two ($C_1$-$C_{18}$)-alkyl radicals,

and

c) 0.1 to 30% by weight of ethylenically unsaturated surface-active urethane derivatives of the formula I,

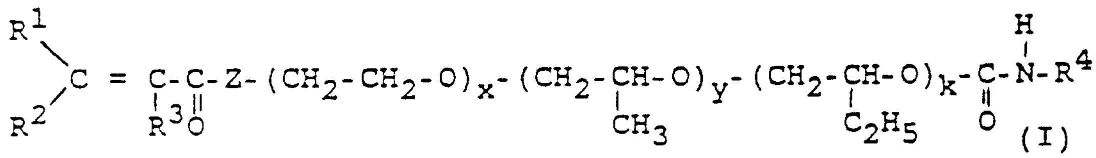

(I)

in which the radicals $R^1$ to $R^4$ and Z and the numbered indices x, y and k have the following meanings:

| | |
|---|---|
| $R^1$, $R^2$, $R^3$, | which can be identical or different, are H, $-CH_3$, -COOH, $-CH_2$-COOH, |
| Z | is oxygen or NH, |
| x, y, k, | which can be identical or different, are 0 to 100, with the proviso that $x + y + k \geqq 2$, |
| $R^4$ | is substituted or unsubstituted ($C_1$-$C_{30}$)-alkyl, substituted or unsubstituted ($C_6$-$C_{10}$)-aryl, substituted or unsubstituted ($C_7$-$C_{30}$)-aralkyl, substituted or unsubstituted ($C_5$-$C_8$)-cycloalkyl, a substituted or unsubstituted 5- to 7-membered heterocycle, |

and

d) 0 to 10% by weight of further ethylenically unsaturated monomers different from a) to c) and having functional radicals from the group comprising -OH,

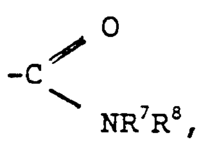

where $R^7$ and $R^8$ can be identical or different and are H, ($C_1$-$C_6$)-alkyl, ($C_2$-$C_8$)-alkoxyalkyl, ($C_5$-$C_7$)-cycloalkyl or ($C_6$-$C_{18}$)-aralkyl, or $R^7$ and $R^8$ together with N form, if desired, a five- to seven-membered heterocyclic ring, preferably ethylenically unsaturated hydroxyalkyl esters or polyalkyleneoxide esters of (meth)acrylic acid, in particular containing 2 to 50 ethylene oxide units and/or 2 to 50 propylene oxide units, in which the terminal OH groups of the ester or polyalkylene glycol ether radicals can also be etherified or esterified, ethylenically unsaturated amides,

and

e) 0 to 5% by weight of ethylenically unsaturated carbonyl compounds, preferably from the group comprising vinyl methyl ketone, acrolein, crotonaldehyde, allyl acetoacetate, acetoacetoxyethyl (meth)acrylate,

and

f) 0 to 5% by weight of ethylenically polyunsaturated or polyfunctional monomers capable of crosslinking, preferably from the group comprising divinylbenzene, diallyl phthalate, butanediol diacrylate, triethylene glycol dimethacrylate, allyl methacrylate, bisphenol A diethylene glycol

dimethacrylate, triallyl cyanurate, methylene-bis(meth)acrylamide, and

g) 0 to 5% by weight of molecular weight regulators from the group comprising dodecylmercaptan, carbon tetrachloride, α-methylstyrene, toluene, bromotrichloromethane, tetrakis(mercaptoacetyl)-pentaerythritol, thioglycolic acid,

and furthermore in the case of emulsion copolymerization adding

h) 0.1 to 10% by weight, relative to the total amount of all monomer units in the copolymer, of emulsifiers and/or if desired, protective colloids, preferably from the group comprising anionic or cationic or zwitter ionic and/or in particular nonionic surfactants and/or protective colloids,

converting the resulting copolymer, if appropriate by partial or complete neutralization, by addition of bases in the case of anionic copolymers and by addition of acids in the case of cationic copolymers into a water-soluble or colloid-water-dispersible copolymer salt and, if desired, obtaining the copolymer product in solid form by eliminating the solvent or dispersant in a conventional manner.

**2.** The process as claimed in claim 1, wherein during the emulsion copolymerization the surface-active comonomer component c) is initially introduced into the water phase.

**3.** The process as claimed in claim 1, wherein the copolymers are present in partially or completely neutralized water-soluble or colloid-water-dispersible form.

**4.** Use of the copolymers prepared as claimed in claims 1, 2 or 3, for the thickening of agueous systems, in particular aqueous dispersions.

**5.** Use of the copolymers prepared as claimed in claims 1, 2 or 3, for the thickening of emulsion paints, gloss emulsion paints, textile printing pastes, paper printing pastes, preparations of biocides, liquid fertilizers, emulsion cleaners, pickling pastes, de-icing agents and cosmetic preparations.

**6.** Use of the copolymers prepared as claimed in claims 1, 2 or 3, for the thickening of aqueous systems, in particular aqueous dispersions, by mixing the copolymers in their unneutralized form capable of salt formation as solutions or dispersions in the required amount with the aqueous system to be thickened, in particular with the dispersion to be thickened, and then partially or completely neutralizing the resulting mixture by addition of bases in the case of anionic copolymers or making it weakly alkaline, or partially or completely neutralizing by addition of acids in the case of cationic copolymers or making it weakly acidic, so that the copolymers are present as water-soluble or colloid-dispersible copolymer salts.

**7.** Use of the copolymers prepared as claimed in claims 1, 2 or 3 as sizing material in the textile industry.

**8.** Use of the copolymers prepared as claimed in claims 1, 2 or 3, as thickeners in amounts of 0.01 to 5 % by weight of copolymer, relative to the aqueous system to be thickened.

**Revendications**
**Revendications pour les Etats contractants suivants : BE, DE, FR, GB, IT**

**1.** Copolymères à base de monomères à insaturation éthylénique, qui contiennent des unités monomères provenant de dérivés d'uréthane tensioactifs ayant des restes carboxy ou carbamide à insaturation éthylénique, et des unités dérivées de monomères salifiables à insaturation éthylénique, et qui ont été préparés par copolymérisation à amorçage radicalaire en solution, en émulsion, en suspension ou en perles, ou leurs solutions ou leurs dispersions aqueuses ou leurs sels ou les solutions ou dispersions des sels, caractérisés en ce que les particules de copolymères sont constituées, en % en masse par rapport à la quantité totale des unités monomères dans le copolymère, de

a) 25 à 85 % en masse de monomères hydrophobes à insaturation éthylénique appartenant au groupe constitué par des esters vinyliques d'acides monocarboxyliques en $C_1$-$C_{18}$, des (méth)-acrylates d'alcools en $C_1$-$C_{22}$, des composés aromatiques vinyliques ayant jusqu'à 18 atomes de carbone, le chlorure de vinyle, l'éthylène, le (méth)acrylonitrile, des diesters de l'acide maléique et/ou fumarique avec des alcools en $C_1$-$C_{22}$, la vinylpyrrolidone, et

b) 1 à 50 % en masse de monomères salifiables à insaturation éthylénique, contenant des restes anioniques fonctionnels du groupe constitué par -COOH, des acides sulfoniques ou des dérivés

d'acides sulfoniques ou des acides phosphoniques ou des dérivés d'acides phosphoniques, des acides carboxyliques du groupe des acides mono- et dicarboxyliques en $C_3$-$C_5$ à insaturation éthylénique et des hémiesters des acides dicarboxyliques avec des alcools linéaires ou ramifiés en $C_1$-$C_8$, ou encore de monomères du groupe constitué par l'acide vinylsulfonique, le méthacrylate de 3-sulfopropyle, l'acide acrylamidométhylpropanesulfonique, l'acide vinylphosphonique, l'acide acrylamidométhylpropanephosphonique ou leurs sels, de préférence les sels de métaux alcalins ou d'ammonium,

ou, à la place des monomères anioniques, des monomères salifiables à insaturation éthylénique contenant des restes cationiques fonctionnels appartenant au groupe constitué par $-NR^5R^6$, où $R^5$ et $R^6$ peuvent être identiques ou différents et représentent H ou un alkyle en $C_1$-$C_{18}$, ou $R^5$ et $R^6$ forment éventuellement ensemble avec N un noyau hétérocyclique de cinq à sept chaînons, ou des amines aliphatiques en $C_3$-$C_{18}$ à insaturation éthylénique primaires ou secondaires avec un reste alkyle en $C_1$-$C_{18}$ ou tertiaires avec deux restes alkyle en $C_1$-$C_{18}$, et

c) 0,1 à 30 % en masse de dérivés d'uréthane tensioactifs à insaturation éthylénique de formule I

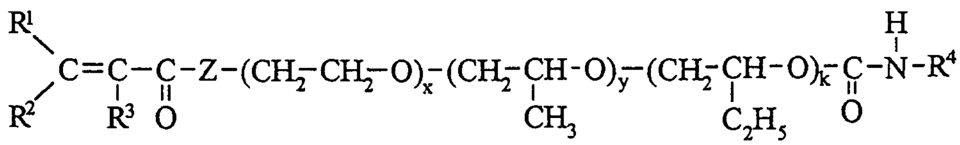 (I)

dans laquelle les restes $R^1$ à $R^4$ et Z et les indices x, y et k ont la signification suivante:

$R^1$, $R^2$, $R^3$, qui peuvent être identiques ou différents, sont H, $-CH_3$, -COOH, $-CH_2$-COOH,

Z est l'oxygène ou NH,

x, y, k, qui peuvent être identiques ou différents, représentent un nombre de 0 à 100, sous réserve que $x + y + k \geq 2$,

$R^4$ représente un alkyle en $C_1$-$C_{30}$ éventuellement substitué, un aryle en $C_6$-$C_{10}$ éventuellement substitué, un aralkyle en $C_7$-$C_{30}$ éventuellement substitué, un cycloalkyle en $C_5$-$C_8$ éventuellement substitué, un hétérocycle de 5 à 7 chaînons éventuellement substitué, et

d) 0 à 10 % en masse d'autres monomères à insaturation éthylénique, différents de a) à c), contenant des restes fonctionnels du groupe constitué par -OH,

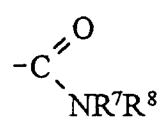,

où $R^7$ et $R^8$ peuvent être identiques ou différents et représentent H, un alkyle en $C_1$-$C_6$, un alcoxyalkyle en $C_2$-$C_8$, un cycloalkyle en $C_5$-$C_7$ ou un aralkyle en $C_6$-$C_{18}$, ou bien $R^7$ et $R^8$ forment éventuellement ensemble avec N un noyau hétérocyclique de cinq à sept chaînons, de préférence des (méth)acrylates d'hydroxyalkyle à insaturation éthylénique ou de poly(oxydes d'alkylène), en particulier avec 2 à 50 unités d'oxyde d'éthylène et/ou 2 à 50 unités d'oxyde de propylène, les groupes OH terminaux des restes ester ou des restes éther de polyalkylèneglycol pouvant aussi être éthérifiés ou estérifiés, des amides à insaturation éthylénique, et

e) 0 à 5 % en masse de composés carbonyle à insaturation éthylénique, appartenant de préférence au groupe constitué par la vinylméthylcétone, l'acroléine, le crotonaldéhyde, l'acétoacétate d'allyle, le (méth)acrylate d'acétoacétoxyéthyle, et

f) 0 à 5 % en masse de monomères à plusieurs insaturations éthyléniques ou polyfonctionnels et aptes à la réticulation, appartenant de préférence au groupe constitué par le divinylbenzène, le phtalate de diallyle, le diacrylate de butanediol, le diméthacrylate de triéthylèneglycol, le méthacrylate d'allyle, le diméthacrylate de bisphénol A-diéthylèneglycol, le cyanurate de triallyle, le méthylènebisméthacrylamide, et

g) 0 à 5 % en masse de régulateurs de masse moléculaire appartenant au groupe constitué par le dodécylmercaptan, le tétrachlorure de carbone, l'α-méthylstyrène, le toluène, le bromotrichlorométhane, le tétrakismercaptoacétylpentaérythritol, l'acide thioglycolique,

et, dans le cas de dispersions aqueuses ou de copolymères isolés à partir de ces dispersions, ces

derniers contiennent en outre

h) 0,1 à 10 % en masse, par rapport à la quantité totale de toutes les unités monomères du copolymère, d'émulsionnants et/ou éventuellement de colloïdes protecteurs, de préférence du groupe constitué par des agents tensioactifs anioniques ou cationiques ou zwitterioniques et/ou en particulier des agents tensioactifs non ioniques et/ou des colloïdes protecteurs.

**2.** Copolymères selon la revendication 1, caractérisés en ce qu'ils sous forme partiellement ou entièrement neutralisée soluble dans l'eau ou formant des dispersions colloïdales dans l'eau.

**3.** Utilisation des copolymères selon les revendications 1 ou 2 pour l'épaississement de systèmes aqueux, en particulier de dispersions aqueuses.

**4.** Utilisation des copolymères selon les revendications 1 ou 2 pour l'épaississement de peintures en dispersion, de vernis brillants en dispersion, de pâtes d'impression pour textiles, de pâtes d'impression pour papier, de préparations de substances actives biocides, d'engrais liquides, de produits de nettoyage en émulsion, de pâtes à décaper, d'agents de dégivrage et de préparations cosmétiques.

**5.** Utilisation des copolymères selon les revendications 1 ou 2 pour l'épaississement de systèmes aqueux, en particulier de dispersions aqueuses, selon laquelle on mélange les copolymères sous la forme salifiable non neutralisée, sous forme de solutions ou de dispersions, en la quantité nécessaire, avec le système aqueux à épaissir, en particulier avec la dispersion à épaissir, puis, dans le cas de polymères anioniques, on neutralise partiellement ou entièrement le mélange obtenu ou on le rend faiblement basique en ajoutant des bases, ou, dans le cas de copolymères cationiques, on neutralise partiellement ou entièrement le mélange obtenu ou on le rend faiblement acide en ajoutant des acides, de sorte que les copolymères se trouvent sous forme de sels de copolymères solubles ou formant des dispersions colloïdales dans l'eau.

**6.** Utilisation des copolymères selon les revendications 1 ou 2 comme agents d'encollage dans l'industrie textile.

**7.** Utilisation des copolymères comme épaississants selon l'une ou plusieurs des revendications 1 à 5 en des quantités introduites de 0,01 à 5 % en masse de copolymère par rapport au système aqueux à épaissir.

**8.** Procédé de préparation de copolymères par copolymérisation à amorçage radicalaire en émulsion ou en solution de monomères copolymérisables à insaturation éthylénique suivie éventuellement d'une neutralisation partielle ou totale des groupes salifiables, et éventuellement de l'isolement des copolymères sous forme déshydratée, caractérisé en ce que, comme comonomères, on copolymérise

a) 25 à 85 % en masse de monomères hydrophobes à insaturation éthylénique appartenant au groupe constitué par des esters vinyliques d'acides monocarboxyliques en $C_1$-$C_{18}$, des (méth)-acrylates d'alcools en $C_1$-$C_{22}$, des composés aromatiques vinyliques ayant jusqu'à 18 atomes de carbone, le chlorure de vinyle, l'éthylène, le (méth)acrylonitrile, des diesters de l'acide maléique et/ou fumarique avec des alcools en $C_1$-$C_{22}$, la vinylpyrrolidone, et

b) 1 à 50 % en masse de monomères salifiables à insaturation éthylénique, contenant des restes anioniques fonctionnels du groupe constitué par -COOH, des acides sulfoniques ou des dérivés d'acides sulfoniques ou des acides phosphoniques ou des dérivés d'acides phosphoniques, des acides carboxyliques du groupe des acides mono- et dicarboxyliques en $C_3$-$C_5$ à insaturation éthylénique et des hémiesters des acides dicarboxyliques avec des alcools linéaires ou ramifiés en $C_1$-$C_8$, ou encore de monomères du groupe constitué par l'acide vinylsulfonique, le méthacrylate de 3-sulfopropyle, l'acide acrylamidométhylpropanesulfonique, l'acide vinylphosphonique, l'acide acrylamidométhylpropanephosphonique ou leurs sels, de préférence les sels de métaux alcalins ou d'ammonium,

ou, à la place des monomères anioniques, des monomères salifiables à insaturation éthylénique contenant des restes cationiques fonctionnels appartenant au groupe constitué par $-NR^5R^6$, où $R^5$ et $R^6$ peuvent être identiques ou différents et représentent H ou un alkyle en $C_1$-$C_{18}$, ou $R^5$ et $R^6$ forment éventuellement ensemble avec N un noyau hétérocyclique de cinq à sept chaînons, ou des amines aliphatiques en $C_3$-$C_{18}$ à insaturation éthylénique primaires ou secondaires avec un reste alkyle en $C_1$-$C_{18}$ ou tertiaires avec deux restes alkyle en $C_1$-$C_{18}$, et

c) 0,1 à 30 % en masse de dérivés d'uréthane tensioactifs à insaturation éthylénique de formule I

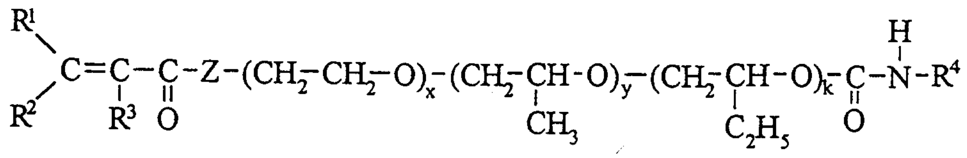 (I)

dans laquelle les restes $R^1$ à $R^4$ et Z et les indices x, y et k ont la signification suivante:
$R^1$, $R^2$, $R^3$, qui peuvent être identiques ou différents, sont H, $-CH_3$ -COOH, $-CH_2$-COOH,
Z est l'oxygène ou NH,
x, y, k qui peuvent être identiques ou différents, représentent un nombre de 0 à 100, sous réserve que $x + y + k \geqq 2$,
$R^4$ représente un alkyle en $C_1$-$C_{30}$ éventuellement substitué, un aryle en $C_6$-$C_{10}$ éventuellement substitué, un aralkyle en $C_7$-$C_{30}$ éventuellement substitué, un cycloalkyle en $C_5$-$C_8$ éventuellement substitué, un hétérocycle de 5 à 7 chaînons éventuellement substitué et

d) 0 à 10 % en masse d'autres monomères à insaturation éthylénique, différents de a) à c), contenant des restes fonctionnels du groupe constitué par -OH,

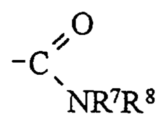 ,

où $R^7$ et $R^8$ peuvent être identiques ou différents et représentent H, un alkyle en $C_1$-$C_6$, un alcoxyalkyle en $C_2$-$C_8$, un cycloalkyle en $C_5$-$C_7$ ou un aralkyle en $C_6$-$C_{18}$, ou bien $R^7$ et $R^8$ forment éventuellement ensemble avec N un noyau hétérocyclique de cinq à sept chaînons, de préférence des (méth)acrylates d'hydroxyalkyle à insaturation éthylénique ou de poly(oxydes d'alkylène), en particulier avec 2 à 50 unités d'oxyde d'éthylène et/ou 2 à 50 unités d'oxyde de propylène, les groupes OH terminaux des restes ester ou des restes éther de polyalkylèneglycol pouvant aussi être éthérifiés ou estérifiés, des amides à insaturation éthylénique, et

e) 0 à 5 % en masse de composés carbonyle à insaturation éthylénique, appartenant de préférence au groupe constitué par la vinylméthylcétone, l'acroléine, le crotonaldéhyde, l'acétoacétate d'allyle, le (méth)acrylate d'acétoacétoxyéthyle, et

f) 0 à 5 % en masse de monomères à plusieurs insaturations éthyléniques ou polyfonctionnels et aptes à la réticulation, appartenant de préférence au groupe constitué par le divinylbenzène, le phtalate de diallyle, le diacrylate de butanediol, le diméthacrylate de triéthylèneglycol, le méthacrylate d'allyle, le diméthacrylate de bisphénol A-diéthylèneglycol, le cyanurate de triallyle, le méthylènebisméthacrylamide, et

g) 0 à 5 % en masse de régulateurs de masse moléculaire appartenant au groupe constitué par le dodécylmercaptan, le tétrachlorure de carbone, l'$\alpha$-méthylstyrène, le toluène, le bromotrichlorométhane, le tétrakismercaptoacétylpentaérythritol, l'acide thioglycolique,

et en outre, dans le cas de la copolymérisation en émulsion, on ajoute

h) 0,1 à 10 % en masse, par rapport à la quantité totale de toutes les unités monomères du copolymère, d'émulsionnants et/ou éventuellement de colloïdes protecteurs, de préférence du groupe constitué par des agents tensioactifs anioniques ou cationiques ou zwitterioniques et/ou en particulier des agents tensioactifs non ioniques et/ou des colloïdes protecteurs,

on transforme éventuellement le copolymère obtenu en un sel de copolymère soluble dans l'eau ou formant des dispersions colloïdales dans l'eau par neutralisation partielle ou totale, et ce, dans le cas de copolymères anioniques, en ajoutant des bases et, dans le cas de copolymères cationiques, en ajoutant des acides, et on prépare éventuellement le produit polymère sous forme solide en éliminant de façon classique le solvant ou l'agent dispersant.

**9.** Procédé selon la revendication 8, caractérisé en ce que, dans la copolymérisation en émulsion, on introduit au préalable le constituant c) comonomère tensioactif dans la phase aqueuse.

**Revendications pour L'Etat contractant suivant : ES**

1. Procédé de préparation de copolymères à base de monomères à insaturation éthylénique, qui contiennent des unités monomères provenant de dérivés d'uréthane tensioactifs ayant des restes carboxy ou carbamide à insaturation éthylénique, et des unités dérivées de monomères salifiables à insaturation éthylénique, par copolymérisation à amorçage radicalaire en émulsion, en suspension, en perles ou en solution de monomères copolymérisables à insaturation éthylénique, suivie éventuellement d'une neutralisation partielle ou totale des groupes salifiables et éventuellement de l'isolement des copolymères sous forme déshydratée, caractérisé en ce que, comme comonomères, on copolymérise par voie radicalaire selon des méthodes classiques

a) 25 à 85 % en masse de monomères hydrophobes à insaturation éthylénique appartenant au groupe constitué par des esters vinyliques d'acides monocarboxyliques en $C_1$-$C_{18}$, des (méth)-acrylates d'alcools en $C_1$-$C_{22}$, des composés aromatiques vinyliques ayant jusqu'à 18 atomes de carbone, le chlorure de vinyle, l'éthylène, le (méth)acrylonitrile, des diesters de l'acide maléique et/ou fumarique avec des alcools en $C_1$-$C_{22}$, la vinylpyrrolidone, et

b) 1 à 50 % en masse de monomères salifiables à insaturation éthylénique, contenant des restes anioniques fonctionnels du groupe constitué par -COOH, des acides sulfoniques ou des dérivés d'acides sulfoniques ou des acides phosphoniques ou des dérivés d'acides phosphoniques, des acides carboxyliques du groupe des acides mono- et dicarboxyliques en $C_3$-$C_5$ à insaturation éthylénique et des hémiesters des acides dicarboxyliques avec des alcools linéaires ou ramifiés en $C_1$-$C_8$, ou encore de monomères du groupe constitué par l'acide vinylsulfonique, le méthacrylate de 3-sulfopropyle, l'acide acrylamidométhylpropanesulfonique, l'acide vinylphosphonique, l'acide acrylamidométhylpropanephosphonique ou leurs sels, de préférence les sels de métaux alcalins ou d'ammonium,

ou, à la place des monomères anioniques, des monomères salifiables à insaturation éthylénique contenant des restes cationiques fonctionnels appartenant au groupe constitué par $-NR^5R^6$, où $R^5$ et $R^6$ peuvent être identiques ou différents et représentent H ou un alkyle en $C_1$-$C_{18}$, ou $R^5$ et $R^6$ forment éventuellement ensemble avec N un noyau hétérocyclique de cinq à sept chaînons, ou des amines aliphatiques en $C_3$-$C_{18}$ à insaturation éthylénique primaires ou secondaires avec un reste alkyle en $C_1$-$C_{18}$ ou tertiaires avec deux restes alkyle en $C_1$-$C_{18}$, et

c) 0,1 à 30 % en masse de dérivés d'uréthane tensioactifs à insaturation éthylénique de formule I

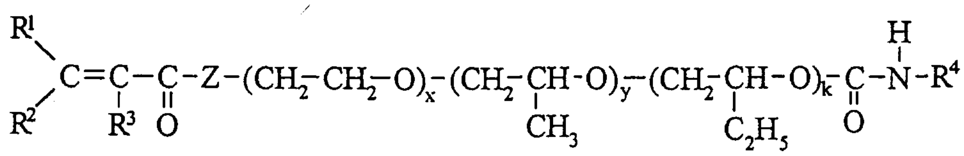

(I)

dans laquelle les restes $R^1$ à $R^4$ et Z et les indices x, y et k ont la signification suivante:

$R^1$, $R^2$, $R^3$, qui peuvent être identiques ou différents, sont H, $-CH_3$, -COOH, $-CH_2$-COOH,

Z est l'oxygène ou NH,

x, y, k qui peuvent être identiques ou différents, représentent un nombre de 0 à 100, sous réserve que $x + y + k \geq 2$,

$R^4$ représente un alkyle en $C_1$-$C_{30}$ éventuellement substitué, un aryle en $C_6$-$C_{10}$ éventuellement substitué, un aralkyle en $C_7$-$C_{30}$ éventuellement substitué, un cycloalkyle en $C_5$-$C_8$ éventuellement substitué, un hétérocycle de 5 à 7 chaînons éventuellement substitué, et

d) 0 à 10 % en masse d'autres monomères à insaturation éthylénique, différents de a) à c), contenant des restes fonctionnels du groupe constitué par -OH,

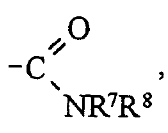

,

où $R^7$ et $R^8$ peuvent être identiques ou différents et représentent H, un alkyle en $C_1$-$C_6$, un alcoxyalkyle en $C_2$-$C_8$, un cycloalkyle en $C_5$-$C_7$ ou un aralkyle en $C_6$-$C_{18}$, ou bien $R^7$ et $R^8$ forment

éventuellement ensemble avec N un noyau hétérocyclique de cinq à sept chaînons, de préférence des (méth)acrylates d'hydroxyalkyle à insaturation éthylénique ou de poly(oxydes d'alkylène), en particulier avec 2 à 50 unités d'oxyde d'éthylène et/ou 2 à 50 unités d'oxyde de propylène, les groupes OH terminaux des restes ester ou des restes éther de polyalkylèneglycol pouvant aussi être éthérifiés ou estérifiés, des amides à insaturation éthylénique, et

e) 0 à 5 % en masse de composés carbonyle à insaturation éthylénique, appartenant de préférence au groupe constitué par la vinylméthylcétone, l'acroléine, le crotonaldéhyde, l'acétoacétate d'allyle, le (méth)acrylate d'acétoacétoxyéthyle, et

f) 0 à 5 % en masse de monomères à plusieurs insaturations éthyléniques ou polyfonctionnels et aptes à la réticulation, appartenant de préférence au groupe constitué par le divinylbenzène, le phtalate de diallyle, le diacrylate de butanediol, le diméthacrylate de triéthylèneglycol, le méthacrylate d'allyle, le diméthacrylate de bisphénol A-diéthylèneglycol, le cyanurate de triallyle, le méthylènebisméthacrylamide, et

g) 0 à 5 % en masse de régulateurs de masse moléculaire appartenant au groupe constitué par le dodécylmercaptan, le tétrachlorure de carbone, l'$\alpha$-méthylstyrène, le toluène, le bromotrichlorométhane, le tétrakismercaptoacétylpentaérythritol, l'acide thioglycolique,

et en outre, dans le cas de la copolymérisation en émulsion, on ajoute

h) 0,1 à 10 % en masse, par rapport à la quantité totale de toutes les unités monomères du copolymère, d'émulsionnants et/ou éventuellement de colloïdes protecteurs, de préférence du groupe constitué par des agents tensioactifs anioniques ou cationiques ou zwitterioniques et/ou en particulier des agents tensioactifs non ioniques et/ou des colloïdes protecteurs,

on transforme éventuellement le copolymère obtenu en un sel de copolymère soluble dans l'eau ou formant des dispersions colloïdales dans l'eau par neutralisation partielle ou totale, et ce, dans le cas de copolymères anioniques, en ajoutant des bases et, dans le cas de copolymères cationiques, en ajoutant des acides, et on prépare éventuellement le produit polymère sous forme solide en éliminant de façon classique le solvant ou l'agent dispersant.

**2.** Procédé selon la revendication 1, caractérisé en ce que, dans la copolymérisation en émulsion, on introduit au préalable le constituant c) comonomère tensioactif dans la phase aqueuse.

**3.** Procédé selon les revendications 1 ou 2, caractérisé en ce que les copolymères sont obtenus sous forme partiellement ou entièrement neutralisée soluble dans l'eau ou formant des dispersions colloïdales dans l'eau.

**4.** Utilisation des copolymères préparés selon les revendications 1,2 ou 3 pour l'épaississement de systèmes aqueux, en particulier de dispersions aqueuses.

**5.** Utilisation des copolymères préparés selon les revendications 1,2 ou 3 pour l'épaississement de peintures en dispersion, de vernis brillants en dispersion, de pâtes d'impression pour textiles, de pâtes d'impression pour papier, de préparations de substances actives biocides, d'engrais liquides, de produits de nettoyage en émulsion, de pâtes à décaper, d'agents de dégivrage et de préparations cosmétiques.

**6.** Utilisation des copolymères préparés selon les revendications 1,2 ou 3 pour l'épaississement de systèmes aqueux, en particulier de dispersions aqueuses, selon laquelle on mélange les copolymères sous la forme salifiable non neutralisée, sous forme de solutions ou de dispersions, en la quantité nécessaire, avec le système aqueux à épaissir, en particulier avec la dispersion à épaissir, puis, dans le cas de polymères anioniques, on neutralise partiellement ou entièrement le mélange obtenu ou on le rend faiblement basique en ajoutant des bases, ou, dans le cas de copolymères cationiques, on neutralise partiellement ou entièrement le mélange obtenu ou on le rend faiblement acide en ajoutant des acides, de sorte que les copolymères se trouvent sous forme de sels de copolymères solubles ou formant des dispersions colloïdales dans l'eau.

**7.** Utilisation des copolymères préparés selon les revendications 1,2 ou 3 comme agents d'encollage dans l'industrie textile.

**8.** Utilisation des copolymères préparés selon les revendications 1,2 ou 3 comme épaississants en des quantités introduites de 0,01 à 5 % en masse de copolymère par rapport au système aqueux à

épaissir.